(19) Europäisches Patentamt
European Patent Office
Office européen des brevets

(11) **EP 4 495 308 A1**

(12) **EUROPEAN PATENT APPLICATION**

(43) Date of publication:
**22.01.2025 Bulletin 2025/04**

(21) Application number: **24188914.6**

(22) Date of filing: **16.07.2024**

(51) International Patent Classification (IPC):
*D04H 1/425* (2012.01)    *D04H 1/4258* (2012.01)
*D04H 1/495* (2012.01)    *D04H 1/498* (2012.01)
*B32B 3/30* (2006.01)    *B32B 5/02* (2006.01)
*B32B 5/26* (2006.01)    *A61F 13/511* (2006.01)
*A61F 13/537* (2006.01)    *A61F 13/514* (2006.01)

(52) Cooperative Patent Classification (CPC):
**D04H 1/498; B32B 3/30; B32B 5/022; B32B 5/26;
D04H 1/425; D04H 1/4258; D04H 1/495;**
A61F 13/51104; A61F 13/5116; A61F 13/5148;
A61F 13/537

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.07.2023 US 202363528174 P**

(71) Applicant: **The Procter & Gamble Company
Cincinnati, OH 45202 (US)**

(72) Inventors:
• WIMALASENA, Nirosha Seth
  Cincinnati, 45202 (US)
• MARSH, Randall Glenn
  Cincinnati, 45202 (US)
• WEISMAN, Paul Thomas
  Cincinnati, 45202 (US)
• XI, Erte
  Cincinnati, 45202 (US)

(74) Representative: **P&G Patent Germany
Procter & Gamble Service GmbH
Sulzbacher Straße 40
65824 Schwalbach am Taunus (DE)**

(54) **NONWOVEN SUBSTRATES AND METHODS OF MAKING THE SAME**

(57) Nonwoven substrates and methods for making nonwoven substrates are provided. The nonwoven substrates have a first material layer containing a first plurality of fibers, a second material layer containing a second plurality of fibers, and a third material layer containing a third plurality of fibers. A first outer surface is formed from the first material layer. The nonwoven substrate has a plurality of elevated regions and a plurality of non-elevated regions disposed on the first outer surface. Fibers of the third plurality of fibers are disposed on the first outer surface in at least some of the plurality of elevated regions.

FIG. 1

EP 4 495 308 A1

## Description

FIELD

The present disclosure is directed to nonwoven substrates having a first material layer, a second material layer, and a third material layer. The present disclosure is also directed to methods of making nonwoven substrates.

BACKGROUND

Nonwoven substrates are often incorporated as various components of consumer products, such as absorbent articles and wet wipes. The application the nonwoven substrate will be used in usually defines characteristics that the nonwoven substrate will be required to exhibit. For example, nonwoven substrates used as topsheets of absorbent articles will typically be required to be soft and liquid permeable. Nonwoven substrates used in wet wipe applications should have good wet strength yet also be soft and able to retain liquid. The desired attributes for a particular application, however, are frequently in conflict with each other - for example, a material that is soft typically has poor wet strength. There is a need for improved nonwoven substrates having a combination of materials that will provide a balance of attributes for use in various consumer products.

SUMMARY

The present disclosure solves one or more of the above problems by providing nonwoven substrates and methods of making nonwoven substrates having a combination of materials and structures that provide desirable attributes for nonwoven substrates employed in various consumer products.

The present disclosure provides, in part, nonwoven substrates comprising a first material layer comprising a first plurality of fibers, a second material layer comprising a second plurality of fibers, and a third material layer comprising a third plurality of fibers. The nonwoven substrates have a first outer surface and a second outer surface opposite the first outer surface. A plurality of elevated regions are disposed on the first outer surface and a plurality of non-elevated regions are disposed on the first outer surface and between the plurality of elevated regions. Fibers of the third plurality of fibers reside on the first outer surface in at least some of the plurality of elevated regions.

The present disclosure provides, in part, methods of manufacturing nonwoven substrates, comprising the steps of depositing a first plurality of fibers on a forming belt, depositing a third plurality of fibers on the first material layer in a face-to-face relationship, depositing a second plurality of fibers on the third material layer and bonding the first material layer, third material layer, and second material layer to form a nonwoven substrate.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Fig. 1 is a cross-section view of a nonwoven substrate of the present disclosure;

Fig. 2 is a plan view of a nonwoven substrate of the present disclosure, first outer surface facing the viewer;

Fig. 3 is a cross-section view of the nonwoven substrate of Fig. 2, taken at line 3-3;

Fig. 4 is a cross-section view of a nonwoven substrate of the present disclosure;

Fig. 5 is a perspective view of a package of nonwoven substrates;

Fig. 6 is a cross-section of the package of Fig. 5, taken through line 6-6 shown in Fig. 5;

Fig. 7 is a plan view of a portion of a package face with an outline of perforations covered by a sticker over-label;

Fig. 8 is a cross section of the portion of the package face of Fig. 7, taken through line 8-8 shown in Fig. 7;

Fig. 9 is a diagrammatic view of an apparatus for performing a process for making nonwoven substrates;

Fig. 10 is a diagrammatic view of an apparatus for patterning a nonwoven substrate;

Fig. 11 is a plan view of a portion of a packaged nonwoven substrate, the package access opening facing the viewer;

Fig. 12 is a cross-section view of the package of Fig. 11, taken through line 12-12 shown in Fig. 11;

Fig. 13 is a cross-section view of a first pouch of a preservative generation system;

Fig. 14 is a plan view of a package with an opening feature flexible film covering the package access opening and a portion of a second pouch of the preservative generation system;

3     **EP 4 495 308 A1**     4

Fig. 15 is a plan view of an example absorbent article in the form of a taped diaper, garment-facing surface facing the viewer, in a flat laid-out state;

Fig. 16 is a plan view of the example absorbent article of Fig. 15, wearer-facing surface facing the viewer, in a flat laid-out state;

Fig. 17 is a plan view of an example absorbent core or an absorbent article;

Fig. 18 is a cross-sectional view, taken about line 18-18, of the absorbent core of Fig. 17; and

Fig. 19 is a cross-sectional view, taken about line 19-19, of the absorbent core of Fig. 17.

## DETAILED DESCRIPTION

Various non-limiting forms of the present disclosure will now be described to provide an overall understanding of the principles of the structure, function, manufacture, and use of the nonwoven substrates and methods of making the same disclosed herein. One or more examples of these non-limiting forms are illustrated in the accompanying drawings. Those of ordinary skill in the art will understand that the nonwoven substrates and methods of making the same described herein and illustrated in the accompanying drawings are non-limiting example forms. The features illustrated or described in connection with one non-limiting form may be combined with the features of other non-limiting forms. Such modifications and variations are intended to be included within the scope of the present disclosure.

### Nonwoven Substrates

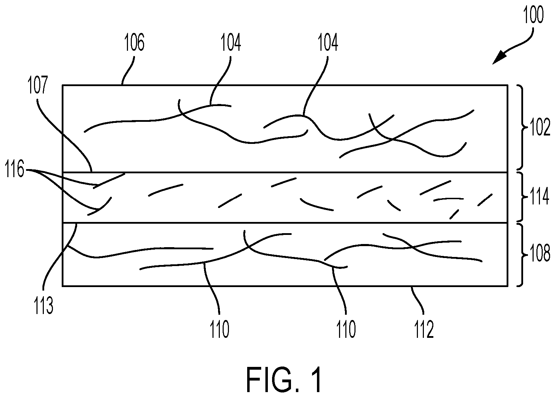

Referring to Fig. 1, which is a cross-section of a nonwoven substrate of the present disclosure, the nonwoven substrates 100 of the present disclosure may comprise a first material layer 102 comprising a first plurality of fibers 104. The first material layer 102 may have a first outer surface 106 and a first interior surface 107. The nonwoven substrates 100 may comprise a second material layer 108 comprising a second plurality of fibers 110. The second material layer 108 may have a second outer surface 112 and a second interior surface 113. The nonwoven substrates 100 of the present disclosure may comprise a third material layer 114 comprising a third plurality of fibers 116.

The first plurality of fibers 104 may comprise or consist of cellulosic fibers. Non-limiting examples of cellulosic fibers include wood pulp, typical northern softwood Kraft, typical southern softwood Kraft, typical CTMP, typical deinked, corn pulp, acacia, eucalyptus, aspen, reed pulp, birch, maple, radiata pine, albardine, esparto, wheat, rice, corn, sugar cane, papyrus, jute, reed, sabia, raphia, bamboo, sisal, kenaf, abaca, cotton, flax, hemp, jute, modified natural cellulosic fibers such as, for example, rayon (including viscose, lyocell, MODAL (a product of Lenzing AG, Lenzing, Austria) and cuprammonium rayon), and combinations thereof. Cellulosic fibers may be consumer-preferred as they may appeal to a desire for natural and/or environmentally friendly products. The cellulosic fibers may be treated or otherwise modified mechanically and/or chemically to provide desired characteristics or may be in a form that is generally similar to the form in which they may be found in nature. Mechanical and/or chemical manipulation of natural fibers does not exclude them from what are considered natural fibers with respect to the present disclosure. The first plurality of fibers 104 may comprise between about 50% and about 100%, between about 60% and about 100%, between about 70% and about 100%, between about 80% and about 100%, or between about 90% and about 100% by weight of the first plurality of fibers of cellulosic fibers, specifically reciting each 1% increment within these ranges and every range formed therein or thereby. The cellulosic fibers may have a linear density of between about 0.5 dtex and about 2.75 dtex, between about 1.0 dtex and about 2.5 dtex, between about 1.25 dtex and about 2.25 dtex, between about 1.45 dtex and about 2.0 dtex, or between about 1.55 dtex and about 1.85 dtex, specifically reciting every 0.1 dtex increment within these ranges and every range formed therein or thereby.

The first plurality of fibers 104 may comprise synthetic fibers. Non-limiting examples of synthetic fibers include polyolefins (polypropylene and polypropylene copolymers, polyethylene and polyethylene copolymers), polyesters, polyamides, polyimide, polylactic acid, polyhydroxyalkanoate, polyvinyl alcohol, ethylene vinyl alcohol, nylon, polyacrylates, and copolymers thereof and mixtures thereof. The polyester may comprise less than 100 ppm antimony, or the polyester may be devoid of antimony. Further, the synthetic fibers may be a single component (i.e., single synthetic material or mixture makes up entire fiber), bi-component (i.e., the fiber is divided into regions, the regions including two or more different synthetic materials or mixtures thereof and may include co-extruded fibers and core and sheath fibers) and combinations thereof. Bi-component fibers may be in a side-by-side, sheath-core, segmented pie, ribbon, or islands-in-the-sea configuration. Bi-component fibers may be used as a component fiber of the structure, and/or they may be present to act as a binder for the other fibers present in the fibrous structure. Any or all of the synthetic fibers may be treated before, during, or after manufacture to change any desired properties of the fibers. The substrate may comprise hydrophilic fibers, hydrophobic fibers, or a combination thereof.

The first plurality of fibers 104 may comprise a combination of low denier and high denier synthetic thermoplastic fibers. The term "denier" as used herein refers to a unit used to indicate the fineness of a filament/fiber. The unit expresses the mass of a filament/fi-

**3**

ber in grams per 9000 meters of length. The low denier thermoplastic fibers may have a denier in the range of from 0.6 dpf (denier per filament) to 1.2 dpf, or from 0.7 dpf to 1.1 dpf, or from 0.8 dpf to 1.1 dpf, or from 0.8 to 1 dpf, or from 0.9 to 1 dpf, specifically reciting every 0.1 dpf increment within these ranges and every range formed therein or thereby. The high denier thermoplastic fibers may have a denier in the range of from 2.2 dpf to 6 dpf, or from 2.5 dpf to 5 dpf or from 2.8 dpf to 4.5 dpf, specifically reciting every 0.1 dpf increment within these ranges and every range formed therein or thereby. The low denier thermoplastic fibers may be various multi-lobal shaped fibers such as trilobal shaped fibers. Other multi-lobal shaped fibers include, but are not limited to, bi-lobal and quattro-lobal shaped fibers. The thermoplastic shaped fibers may also include delta shaped, concave delta shaped, crescent shaped, oval shaped, star shaped, trapezoid shaped, square shaped, diamond shaped, U-shaped, H-shaped, C-shaped, V-shaped, or other suitable shaped fibers or any combinations thereof. The low denier thermoplastic shaped fibers may include any combinations of the above mentioned shaped fibers. The thermoplastic shaped fibers may be solid or hollow fibers.

The first plurality of fibers 104 may comprise from about 20% to about 90%, by weight of the total amount of fibers, of low denier thermoplastic fibers and from about 10% to about 80%, by weight of the total amount of fibers, of high denier thermoplastic fibers. In another form, where the first plurality of fibers also comprises cellulosic fibers, the first plurality of fibers may comprise from about 20% to about 90%, by weight of the thermoplastic fiber-portion of the first plurality of fibers, of low denier thermoplastic fibers and from about 10% to about 80%, by weight of the thermoplastic fiber-portion of the first plurality of fibers, of high denier thermoplastic fibers. The first plurality of fibers 104 may comprise at least 0.2%, by weight of the plurality of fibers, of an opacifying agent, such as, for example, titanium dioxide.

The first plurality of fibers may comprise, or consist of, staple fibers having an average fiber length between about 20 mm and about 80 mm, between about 25 mm and about 75 mm, between about 30 mm and about 70 mm, or between about 35 mm and about 65 mm, specifically reciting every 1.0 mm increment within these ranges and every range formed therein or thereby. In a form, the first plurality of fibers 104 may comprise staple lyocell fibers. In a form, the first plurality of fibers 104 may consist of staple lyocell fibers.

Referring again to Fig. 1, the first material layer 102 may comprise, or consist of, the first plurality of fibers 104. In another form, the second material layer 108 may comprise, or consist of, the first plurality of fibers 104. The first material layer 102 and/or the first plurality of fibers 104 may make up from about 5% to about 45%, between about 10% and about 40%, or between about 15% and about 35% by weight of the nonwoven substrate. In one embodiment, the first plurality of fibers 104 makes up 30% by weight of the nonwoven substrate. The first material layer may have a basis weight of between about 10 gsm (grams per square meter) and about 30 gsm, between about 15 gsm and about 25 gsm, or between about 18 gsm and about 22 gsm, specifically reciting every 1 gsm increment within these ranges and every range formed therein or thereby. The first material layer 102 may comprise a plurality of staple lyocell fibers. The first material layer 102 may consist of a plurality of staple lyocell fibers. The first material layer 102 may comprise a first outer surface 106 of the nonwoven substrate 100. The first plurality of fibers 104 may be devoid of thermoplastic material. Furthermore, the first material layer 102 and/or the first plurality of fibers 104 may be free of petroleum-based materials.

The second plurality of fibers 110 may be formed from the same blend of fibers as the first plurality of fibers 104, or the second plurality of fibers 110 may comprise fibers or a blend of fibers that are different than those found in the first plurality of fibers 104. The second plurality of fibers 110 may comprise, or consist of, cellulosic fibers and/or synthetic fibers as described herein with respect to the first plurality of fibers 104. Referring again to Fig. 1, the second material layer 108 may comprise, or consist of, the second plurality of fibers 110. In another form, the first material layer 102 may comprise, or consist of, the second plurality of fibers 110. The second material layer 108 and/or the second plurality of fibers 110 may make up from about 5% to about 45%, between about 10% and about 40%, or between about 15% and about 35% by weight of the nonwoven substrate. In one embodiment, the second plurality of fibers 110 makes up 30% by weight of the nonwoven substrate. The second material layer 108 may have a basis weight of between about 10 gsm and about 30 gsm, between about 15 gsm and about 25 gsm, or between about 18 gsm and about 22 gsm, specifically reciting every 1 gsm increment within these ranges and every range formed therein or thereby. The second material layer 108 may have the same basis weight as the first material layer 102, or the second material layer 108 may have a different basis weight than the first material layer 102. The second material layer 108 may comprise a second outer surface 112 of the nonwoven substrate 100 opposite the first outer surface 106.

The first and/or the second material layers 102, 108 or the first and/or second plurality of fibers 104, 110 may have a fluid retention value (FRV) of less than 0.95 g/g, less than 0.92 g/g, less than 0.9 g/g, less than 0.85 g/g, or less than 0.8 g/g as measured according to the Fluid Retention Value Test Method disclosed herein. The first and/or the second material layers 102, 108 or the first and/or second plurality of fibers 104, 110 may have a fluid retention value that is different from the fluid retention value of the third material layer 114 and/or the third plurality of fibers 116. The first and/or the second material layers 102, 108 or the first and/or second plurality of fibers 104, 110 may have a fluid retention value that is less than the fluid retention value of the third material layer 114 and/or the third plurality of fibers 116.

The third plurality of fibers 116 may comprise, or consist of, cellulosic fibers. The cellulosic fibers of the third plurality of fibers 116 may be the same as those in the first plurality of fibers 104 and/or the second plurality of fibers 110, or they may be different. The third plurality of fibers 116 may comprise, or consist of, pulp fibers, also described herein as wood pulp fibers. Pulp fibers may include chemical pulps, such as Kraft, sulfite, and sulfate pulps, as well as mechanical pulps including, for example, groundwood, thermomechanical pulp and chemically modified thermomechanical pulp. Pulps derived from both angiosperm (flowering) trees (hereinafter, also referred to as "hardwood") and gymnosperm (coniferous) trees (hereinafter, also referred to as "softwood") may be utilized. The hardwood and softwood fibers can be blended, or alternatively, can be deposited in layers to provide a stratified web. Pulp fibers may be derived from recycled paper, such as newsprint, paperboard, and other sources of post-industrial and/or post-consumer pulp fiber waste.

The third plurality of fibers 116 may comprise a blend of long or medium-length pulp fibers and short pulp fibers. Generally, long and medium-length fibers tend to be larger and coarser, providing desirable texture and absorption characteristics, while short fibers tend to be finer and softer, enhancing opacity of the structure and adding tactile softness. Including short pulp fibers as a portion of the fiber blend may be beneficial for controllably including consolidated masses of fibers in the blend. A blend of softwood pulp fibers (medium-length) and hardwood pulp fibers (short) may be used. The softwood and hardwood pulp fibers, or medium-length and short fibers, may be included in a weight ratio of 20:80 to 90: 10. For purposes herein, it may be desired that the weight ratio of softwood fibers to hardwood fibers, or weight ratio of medium-length fibers to short fibers, be from 60:40 to 90:10, more preferably 65:35 to 85:15, and still more preferably 70:30 to 80:20 in the structure. In a more particular example within these ranges, the softwood pulp fibers may be SSK (southern softwood kraft) pulp fibers. In another more particular example within these ranges, the hardwood pulp fibers may be birch, aspen or eucalyptus pulp fibers. In a still more particular example within these ranges, the softwood pulp fibers may be SSK pulp fibers, and the hardwood pulp fibers may be birch, aspen or eucalyptus pulp fibers. Aspen, birch or eucalyptus pulp fibers may be desirable for their fineness, shortness, and softness, which contribute to enhancing opacity and softness of the fibrous web structure, and eucalyptus pulp may be particularly preferred for these characteristics. In addition to the various wood pulp fibers, other cellulosic fibers such as cotton linters, rayon, lyocell, viscose and bagasse may be used. Other sources of cellulose in the form of fibers or materials capable of being spun into fibers include grasses and grain sources. The third plurality of fibers 116 may have an average fiber length of between about 0.5 mm and about 25 mm, between about 0.5 mm and about 20 mm, or between about 0.5 mm and about 17 mm, specifically reciting each 0.1 mm increment within these ranges and every range formed therein or thereby. The fibers forming the third material layer 114 may have a shorter average fiber length than the fibers forming the first material layer 102 and/or the second material layer 108.

At least a portion of individual fibers of the third plurality of fibers 116 may be bonded to one another. A portion of the individual fibers of the third plurality of fibers 116 may be covalently bonded to one another. Covalent bonding may be achieved by the inclusion of one or more binding agents and/or wet strength agents. Binding agents may be selected from the group consisting of chitosan, modified starch, cellulose derivatives, in particular blends of carboxymethylcellulose and citric acid, protein based binders, such as casein, polyamide-epichlorohydrin (PAE) resins, and combinations of two or more thereof. Wet strength agents may include chitosan, modified starch, cellulose derivatives such as cellulose ethers and cellulose esters, such as methylcellulose, ethylcellulose, hydroxyethyl cellulose, hydroxypropyl cellulose, carboxymethyl cellulose, cellulose acetate, polyamide-epichlorohydrin (PAE) resins, and combinations thereof.

A portion of the individual fibers of the third plurality of fibers 116 may be hydrogen bonded to one another. As opposed to covalent bonding through binding agents and/or wet strength agents, hydrogen bonding may occur between hydroxyl groups of cellulose and hemicellulose fibers of the pulp fibers of the third plurality of fibers without additional additives or binder agents. Hydrogen bonds are also weaker than covalent bonds and may be broken upon the application of mechanical force - such as hydroentanglement - to allow the fibers to reorganize and form different structures within the nonwoven substrate that may not be possible when the fibers are covalently bonded to one another. The third plurality of fibers 116 may be free of binder agents and wet strength agents.

The third plurality of fibers 116 may be incorporated into the nonwoven substrate 100 as a preformed batt of material, such as, for example, a preformed tissue layer. At least a portion of the individual fibers of the third plurality of fibers 116 may be hydrogen bonded to one another and may be free of binder agents, wet-strength agents, and the like.

Referring again to Fig. 1, the third material layer 114 may comprise, or consist of, the third plurality of fibers 116. The third material layer 114 or the third plurality of fibers 116 may make up between about 10% and about 65%, between about 20% and about 50%, between about 25% and about 45%, or between about 30% and about 42% by weight of the nonwoven substrate. In one embodiment, the third plurality of fibers 116 makes up 40% by weight of the nonwoven substrate. The third material layer 114 may have a basis weight of between about 10 gsm and about 45 gsm, between 15 gsm and about 40 gsm, between about 18 gsm and bout 35 gsm, or between about 22 gsm and about 32 gsm, specifically reciting each 1 gsm increment within these ranges and every range formed therein or thereby.

The third material layer and/or the third plurality of fibers may have a fluid retention value (FRV) of greater than 0.98 g/g, greater than 1.0 g/g, greater than 1.03 g/g, or greater than 1.05 g/g, according to the Fluid Retention Value Test Method disclosed herein. The third material layer 114 and/or the third plurality of fibers 116 may have a fluid retention value different than the fluid retention value of the first and/or the second material layers 102, 108 or the first and/or second plurality of fibers 104, 110. The third material layer 114 and/or the third plurality of fibers 116 may have a fluid retention value greater than the fluid retention value of the first and/or the second material layers 102, 108 or the first and/or second plurality of fibers 104, 110. Without wishing to be bound by theory, it is believed that the differential fluid retention values of the first and/or second material layers and the third material layer may provide several benefits to the nonwoven substrate when used as a wet wipe. For example, the third material layer, which may be disposed between the first and second material layers, may act to store excess liquid that may improve the cleaning effectiveness of the nonwoven substrates of the present disclosure.

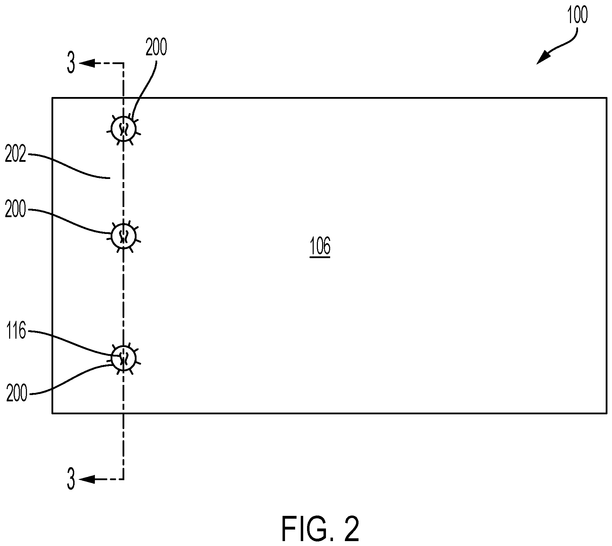

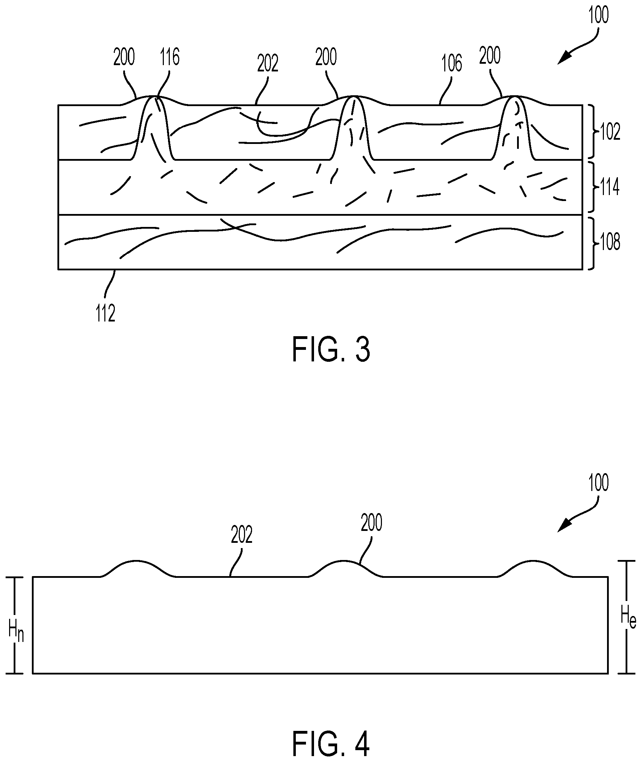

As shown in Fig. 2, the nonwoven substrate 100 of the present disclosure may comprise a plurality of elevated regions 200. The plurality of elevated regions 200 may be disposed on the first outer surface 106 (as shown in Figs. 2 and 3), the second outer surface 112, or both the first outer surface 106 and the second outer surface 112. The nonwoven substrate 100 may also comprise a non-elevated region 202 disposed substantially between the plurality of elevated regions 200. The non-elevated region 202 may be a continuous, interconnecting region, or may be a plurality of discrete regions. The non-elevated ration 202 may substantially surround each of the individual elevated regions comprising the plurality of elevated regions 200. Fibers of the third plurality of fibers 116 may be disposed on the first outer surface 106 in at least some of the plurality of elevated regions 200. In other words, the first outer surface 106 of the nonwoven substrate 100 may be formed at least partially of a portion of the first plurality of fibers 102 and a portion of the third plurality of fibers 116. Where the second outer surface 112 of the nonwoven substrate 100 also comprises a plurality of elevated regions 200, fibers of the third plurality of fibers 116 may be disposed on the second outer surface 112 in at least some of the plurality of elevated regions 200. As discussed further herein with regard to methods of making nonwoven substrates of the present disclosure, at least some fibers of the third plurality of fibers 116 may be disposed on the first outer surface 106 and or the second outer surface 112 through application of hydraulic energy in the form of high pressure streams of fluid impacting the layered structure of the nonwoven substrate and causing at least some of the third plurality of fibers 116 to penetrate into the first material layer 102 and/or the second material layer and reside on the first outer surface 106 and/or second outer surface 112. It is believed that disposition of fibers of the third plurality of fibers 116 on the first outer surface 106 and/or the second outer surface 112 in some of the elevated regions 200 improves the cleaning performance of the nonwoven substrate without having the expected negative impact on softness. It is thought that the retention of softness is due to the controlled exposure of the third plurality of fibers 116 only at the elevated regions, while the third plurality of fibers 116 remains sandwiched between the first material layer 102 and the second material layer 108 in the non-elevated regions 202. Disposition of portions of the third plurality of fibers 116 on the first and/or second outer surface 106, 112 of the nonwoven substrate 100 is also believed to allow a greater amount of fluid retained by the third plurality of fibers 116 (due to the greater fluid retention properties of the third plurality of fibers) to reach the outer surface of the nonwoven substrate to enhance cleaning performance of the nonwoven substrates of the present disclosure.

Referring now to Fig. 3, which is a cross-sectional view of the nonwoven substrate 100 of Fig. 2 taken at line 3-3, at least some of the fibers of the third plurality of fibers 116 may be singularized and intertwined with fibers of the first plurality of fibers 104 in at least some of the elevated regions 200. In other words, a portion of the third material layer 114 may be intermixed with a portion of the first material layer 102 and/or the second material layer 108 within the elevated regions 200, with some of the individual fibers of the third plurality of fibers 116 being singularized - unbonded and/or otherwise disengaged from the cohesive structure of the third material layer 114 - and disposed on the first outer surface 106 and/or the second outer surface 112. The third plurality of fibers 116 may be disposed substantially between the first material layer 102 and the second material layer 108 in the non-elevated region(s) 202. At least some of the fibers of the third plurality of fibers 116 may remain bonded to one another in the non-elevated region(s) 202 of the nonwoven substrates 100 of the present disclosure. In another form, substantially all of, or all of, the individual fibers of the third plurality of fibers 116 may not be bonded to one another and may be singularized.

The nonwoven substrates 100 of the present disclosure may be non-homogenous. As shown for example in Fig. 3, regions of the nonwoven substrates 100 may be layered, exhibiting discrete layers of pluralities of fibers. Non-elevated regions 202 may have discrete layers of different pluralities of fibers - for example a first plurality of fibers 102, a second plurality of fibers 108, and a third plurality of fibers 114 as shown in the non-elevated regions of Fig. 3.

As further shown in Fig. 3, the second outer surface 112 of the nonwoven substrates of the present disclosure may be substantially free of the third plurality of fibers 116. For example, where the third plurality of fibers 116 comprise or consist of natural fibers, such as pulp fibers, the second outer surface 112 may be substantially free of the natural fibers. The second material layer 108 may be substantially free of the third plurality of fibers 116 that form, at least in-part, the third material layer 114. In other words, the second material layer 108 and the third material layer 114 may be discrete layers, substantially free of fibers from another material layer.

Referring to Fig. 4, the plurality of elevated regions 200 of the nonwoven substrates 100 of the present disclosure may have a height ($H_e$) greater than the height of the nonwoven substrates 100 in the non-elevated regions ($H_n$). Height of the various regions of the nonwoven substrates 100 of the present disclosure may be measured by a variety of available methods, including microscopy or use of an electronic caliper gauge. The elevated regions 200 of the nonwoven substrates 100 may have a greater basis weight as compared to the non-elevated region 202. The elevated regions 200 of the nonwoven substrates 100 may have a lower density as compared to the non-elevated region 202. The elevated regions 200 of the nonwoven substrates may comprise a greater proportion of the third plurality of fibers 116 as compared to the non-elevated regions 202. Without wishing to be bound by theory, it is believed that the process of creating the plurality of elevated regions in the nonwoven substrates of the present disclosure - such as hydraulic patterning, for example - reorganize and concentrate a portion of the third plurality of fibers in the plurality of elevated regions. Concentrating the third plurality of fibers within the elevated regions of the nonwoven substrates of the present disclosure may allow for fibers with greater fluid retention ability, such as pulp fibers, to hold a greater amount of liquid in the elevated regions as compared to the non-elevated regions. When a liquid is applied to the nonwoven substrates of the present disclosure, as in a wet wipe application for example, the liquid may concentrate in elevated regions of the nonwoven substrates where there is a higher concentration of absorbent material. This concentration of liquid in the elevated regions of the nonwoven substrate may provide an improved user cleaning experience, with increased cleaning liquid at the portions of the nonwoven substrate that are most in contact with the surface to be cleaned.

Referring again to Fig. 3, while a portion of the third plurality of fibers 116 may be disposed on the first outer surface 106 and/or the second outer surface 112, this portion of the third plurality of fibers 116 may be interconnected with the remainder of the third plurality of fibers 116 that comprise the third material layer 114. This interconnection of the third plurality of fibers 116 within the third material layer 114 may allow for distribution or redistribution of liquid throughout the entire third material layer 114 through capillary action. This redistribution of liquid may allow for the transmission of liquid originally applied to a non-elevated 202 portion of the nonwoven substrate to move from the higher density non-elevated regions 202 and to concentrate in the lower density plurality of elevated regions 200.

Nonwoven substrates of the present disclosure may be formulated without inclusion of components derived from petroleum (such as plastics with petroleum or petroleum-derived components or precursor materials); such that the nonwoven substrates are relatively rapidly dispersible when immersed in water; and such that they are biodegradable, which may contribute to making them less of a burden on water treatment and/or waste management resources. The nonwoven substrates of the present disclosure may contain less than 10 percent by weight, more preferably less than 5 percent by weight, and even more preferably substantially no, petroleum, petroleum derivatives and/or plastics with petroleum or petroleum-derived components or precursor materials.

Further, materials manufactured from or containing cellulose pulp, such as wood pulp, may be recyclable. Nonwoven substrates of the present disclosure comprising or consisting of cellulosic material, including pulp, may be recyclable in conventional paper recycling operations.

The extent of recyclability of the nonwoven substrates of the present disclosure may be expressed as recyclable percentage. It is believed that nonwoven substrates contemplated herein may be manufactured so as to exhibit recyclable percentages of at least 70 percent, more preferably at least 80 percent and even more preferably at least 90 percent; or from about 70 percent to about 99.9 percent, more preferably from about 80 percent to about 99.9 percent, or even more preferably from about 90 percent to about 99.9 percent, including all values within these ranges and any sub-ranges created thereby. It is believed that in some examples, nonwoven substrates contemplated herein may be manufactured to exhibit a recyclable percentage of from about 95 percent to about 99.9 percent, more preferably from about 97 percent to about 99.9 percent, or most preferably from about 98 percent to about 99.9 percent, including all values within these ranges and any sub-ranges created thereby. The recyclable percentage of the nonwoven substrates of the present disclosure can be determined via test PTS-RH:021/97 (Draft Oct. 2019) under category II, as performed by Papiertechnische Stiftung located at Pirnaer Strasse 37, 01809 Heidenau, Germany (herein, "recyclability test"). It is believed that in some examples, nonwoven substrates contemplated herein may be manufactured so as to exhibit an overall "pass" test outcome determined by application of the recyclability test.

Further, it is believed that the nonwoven substrates contemplated herein may be manufactured such that, when subjected to biodegradation testing under ASTM D-6691 available at the date of filing of this application (herein, "marine biodegradability test"), may exhibit substantial marine biodegradation within a given time period (*e.g.,* 180 days, 200 days, 365 days, 400 days, 1 year, 2 years, 3 years, 4 years, or 5 years). It is believed that in some examples, the nonwoven substrates contemplated herein may be manufactured so as to exhibit at least 30 percent, more preferably at least 50 percent, more preferably at least 75 percent, even more preferably at least 90 percent, or most preferably substantially complete biodegradation in a marine environment within about 400 days when tested using the marine biodegradability test.

Even further, it is believed that nonwoven substrates of the present disclosure may be manufactured such that, when subjected to biodegradation testing under ASTM D-5511 available at the date of filing of this application (herein, "landfill biodegradability test"), may exhibit substantial landfill or composting environment biodegradation within a given time period (e.g., 180 days, 200 days, 365 days, 400 days, 1 year, 2 years, 3 years, 4 years, or 5 years). It is believed that in some examples, nonwoven substrates contemplated herein may be manufactured so as to exhibit at least 30 percent, more preferably at least 50 percent, more preferably at least 75 percent, even more preferably at least 90 percent, or most preferably substantially complete total biodegradation in a landfill environment within about 400 days when tested using the landfill biodegradability test.

Consumer Products

The nonwoven substrates of the present disclosure may form a portion of, or be packaged as, a consumer product. The nonwoven substrates may, for example, be packaged as a single nonwoven substrate or as a plurality of individual nonwoven substrates, such as towels or dry wipes. A liquid may be applied to a single nonwoven substrate or a plurality or stack of individual nonwoven substrates, and the consumer product may take the form of wet wipes.

The liquid portion of a wet wipe, herein referred to as a lotion, may comprise various chemical constituents that carry out functions such as cleaning, buffering, and preservation. The lotion may assist in the cleaning function of a wet wipe by, for example, solubilizing and clearing away dirt, fecal, and/or urine residue from the skin of a user. It is believed that, due to certain structural elements of the nonwoven substrates of the present disclosure - such as the disposal of a portion of the third plurality of fibers on an outer surface of the substrate - and certain functional properties of the nonwoven substrates of the present disclosure - such as the differential fluid retention values of the first and second material layers versus the third material layer - wet wipes comprising the nonwoven substrates of the present disclosure exhibit improved cleaning properties over nonwoven substrates not exhibiting these structures and functional properties.

The wet wipes described herein may comprise a lotion. The lotion may comprise at least 90% water, at least 95% water, or from about 95% water to about 99.9% water, from about 97% water to about 99.9% water, or from about 98.2% water to about 99.9% water, by weight of the lotion, specifically reciting every 0.1% increment within these ranges and any ranges formed therein or thereby, according to the Water Content Test Method described herein. A wet wipe lotion comprising water within the above-specified ranges is expected to be gentle on the skin, especially when the wet wipe is employed to cleanse delicate areas of the body.

Lotion Preservative System: The lotions of the present disclosure may comprise a preservative system. A preservative system may be understood to be a synthetic or natural compound, or a combination of compounds, that may be effective in reducing or preventing the growth of microorganisms, thus enabling a longer shelf life for a package of nonwoven substrates, such as wet wipes (opened or not opened). The preservative system may also create an environment that reduces or inhibits growth of microorganisms when the lotion is transferred to the skin during the wiping process. The preservative system may be especially useful when a wet wipe lotion comprises a high concentration of water, such as, for example, greater than about 95% of the lotion formulation.

The spectrum of activity of the preservative systems disclosed herein may include bacteria, molds, and/or yeast. One of, or every, such microorganism may be killed by a preservative system of the present disclosure. Another mode of action of the preservative systems may be the reduction of the growth rate of the microorganisms without active killing.

The preservative systems of the present disclosure may comprise a natural compound or compounds. Natural compounds may be compounds derived from plants and/or animals, or from natural processes of plants and/or animals, such as fermentation, for example. Natural compounds may be, or may be perceived by consumers to be, gentler on the skin of a user as compared to man-made compounds. Natural compounds may comprise compounds derived from plants and/or animals without any post-harvest treatment steps, as well as those receiving post-harvest treatment steps, such as neutralization reactions to form a salt, for example. The lotions of the present disclosure may comprise a preservative system comprising a natural preservative or preservatives. The lotion may comprise a 100% plant-based preservative system. The preservative system may consist essentially of natural compounds. The preservative system may consist of natural compounds.

The lotion preservative systems of the present disclosure may comprise benzoic acid and/or a salt thereof. Benzoic acid is a carboxylic acid that occurs naturally in many plant and animal species. The lotions of the present disclosure may comprise at least 0.1%, at least 0.14%, at least 0.17%, or from about 0.1% to about 0.6%, from about 0.1% to about 0.4%, from about 0.1% to about 0.3%, from about 0.1% to about 0.25%, from about 0.14% to about 0.4%, from about 0.14% to about 0.3%, from about 0.17% to about 0.3%, or from about 0.17% to about 0.25% of benzoic acid, by weight of the lotion, in the form of benzoic acid and/or a salt thereof, specifically reciting every 0.01% increment within these ranges and any ranges formed therein or thereby. The lotion may comprise sodium benzoate. The lotion may comprise benzoic acid and sodium benzoate.

The lotion preservative systems of the present disclosure may comprise succinic acid and/or a salt thereof. Succinic acid is a dicarboxylic acid that is found in many plant and animal sources and may also be synthesized through fermentation by certain microorganisms. Succinic acid is used in the food industry as an acidity regulator and a flavoring agent. Succinic acid has unexpectedly been found to provide hostility against microorganisms in lotions with a high water content. The lotions of the present disclosure may comprise at least 0.15%, at least 0.2%, at least 0.25%, from about 0.15% to about 0.6%, or from about 0.1% to about 0.4%, from about 0.1% to about 0.3%, from about 0.2% to about 0.6%, from about 0.2% to about 0.45%, from about 0.2% to about 0.4%, from about 0.25% to about 0.4%, or from about 0.25% to about 0.35% of succinic acid, by weight of the lotion, in the form of succinic acid and/or a salt thereof, specifically reciting every 0.01% increment within these ranges and any ranges formed therein or thereby. The lotion may comprise sodium succinate. The lotion may comprise succinic acid and sodium succinate.

The lotion preservative systems of the present disclosure may comprise phytic acid and/or a salt thereof. Phytic acid is an organic acid that occurs naturally, for example, in many legumes, grains, and the bran of cereals. The lotions of the present disclosure may comprise at least 0.02%, at least 0.04%, at least 0.06%, or from about 0.02% to about 0.2%, from about 0.02% to about 0.15%, from about 0.02% to about 0.1%, from about 0.04% to about 0.2%, from about 0.04% to about 0.15%, from about 0.04% to about 0.1%, from about 0.06% to about 0.1%, or from about 0.06% to about 0.08% of phytic acid, by weight of the lotion, in the form of phytic acid and/or a salt thereof, specifically reciting every 0.01% increment within these ranges and any ranges formed therein or thereby. The lotion may comprise sodium phytate. The lotion may comprise phytic acid and sodium phytate.

The lotions of the present disclosure may be free of surfactants. The selection of compounds making up the preservative system of the lotions described herein may be effective in reducing or inhibiting the growth of microorganisms, such as bacteria, yeast, and mold, without the need for surfactants to stabilize the preservative system components in the aqueous medium of the lotion. Many surfactants used in wet wipe applications comprise fatty acids and/or are derived from plant-based oils, such as sorbitan caprylate, polysorbate-20, and PEG-40 hydrogenated castor oil. Such surfactants may contribute a fatty odor to the lotion. This fatty odor may be exacerbated if the lotion has a low pH, example below 4.0, due to acid-catalyzed hydrolysis of fatty esters and resulting in generation of free fatty acids over time. Fatty acids may have an odor that is detectable at very low concentrations to the human nose. When assembled into a lotion, even a small amount of fatty acid-containing materials can result in a final formulation that has a characteristic odor that may be noticeable and undesirable to consumers. The lotions of the present disclosure may be free of fatty acid-containing compounds.

Lotion Buffering System: The lotions of the present disclosure may comprise a pH buffering system. The pH buffering system may be present at from about 0.3% to about 1%, from about 0.4% to about 1%, from about 0.5% to about 0.85%, or from about 0.55% to about 0.75%, by weight of the lotion, specifically reciting every 0.01% increment within these ranges and any ranges formed therein or thereby. The pH buffering system may help prevent or reduce the growth of, or kill, bacteria, mold, and/or yeast by, for example, maintaining a pH of the lotion that is hostile to the growth of bacteria, mold, and/or yeast, or by maintaining a pH that makes the bacteria, mold, and/or yeast more susceptible to the preservative system. The pH buffering system may be a citrate-citric acid buffering system at a pH of less than 5. The pH buffering system may be a citrate-citric acid buffering system at a pH of less than 4. The pH buffering system may be a sodium citrate-citric acid buffering system at a pH of less than 5. The pH buffering system may be a sodium citrate-citric acid buffering system at a pH of less than 4. The pH buffering system may comprise sodium citrate dihydrate and citric acid anhydrous. The lotions of the present disclosure may have a pH from about 3.4 to about 6, from about 3.5 to about 5, from about 3.6 to about 4.8, from about 3.6 to about 4.4, from about 3.6 to about 4.2, or from about 3.8 to about 4.2, specifically reciting every 0.1% increment within these ranges and any ranges formed therein or thereby.

The Equivalence Value of a lotion may be an indication of the lotion's resistance to an increase in pH. A lotion having a higher Equivalence Value may be desired because it may help to protect a baby's skin against irritation caused by feces and urine residue that remains after cleaning. The proteases in feces that may damage the skin can start to become active around pH 5 and can reach peak activity between pH 7 to 8. Urea in urine may be converted to ammonia by bacteria on the skin, elevating the pH and making the fecal proteases more active. By depositing a lotion with a higher Equivalence Value on the skin, an acidic buffer may be created that resists the rise in pH that may otherwise occur over time and/or when trace amounts of feces and urine are present. By resisting the rise in pH, a lotion with a higher Equivalence Value may protect the skin from damage caused by fecal proteases and reduce the likelihood that rash will occur between diaper changes. The lotions of the present disclosure may have an Equivalence Value from about 25 to about 100, from about 30 to about 95, from about 55 to about 90, from about 65 to about 90, from about 60 to about 80, from about 62 to about 75, or from about 65 to about 70, specifically reciting every 1.0 unit increment within these ranges and any ranges formed therein or thereby, according to the Buffering Capacity Test Method described herein.

Lotion Optional Ingredients: The lotions of the present disclosure may include various optional ingredients, such as emollients, opacifying agents, film-formers, soothing agents, skin protectants, medically active ingredients, healing actives, and the like, such as described in U.S. Patent Nos. 7,666,827; 7,005,557; and 8,221,774.

The lotions of the present disclosure may comprise an emollient or emollients. Emollients may (1) hydrate soil residues (for example, fecal residues or dried urine residues or menses residues), thus enhancing their removal from the skin, (2) hydrate the skin, thus reducing its dryness and irritation, (3) protect the skin from later irritation (for example, caused by the friction of an absorbent article) as the emollient is deposited onto the skin and remains at its surface as a thin protective layer, and/or (4) provide a desired sensory feel to the lotion and/or the skin.

The lotions of the present disclosure may comprise one or more rheology modifiers. A rheology modifier may help to stabilize the lotion by, for example, reducing or preventing coalescence of droplets of the hydrophobic materials in the composition. Non-limiting examples of rheology modifiers include, but are not limited to, hydrocolloids, including natural gums, such as xanthan gum. The lotions of the present disclosure may comprise from about 0.01% to about 0.1%, from about 0.03% to about 0.08%, from about 0.05% to about 0.07%, or about 0.06% of a rheology modifier, by weight of the lotion, specifically reciting every 0.01% increment within these ranges and every range formed therein or thereby.

The lotions of the present disclosure may comprise a fragrance and/or a perfume. The fragrance and/or perfume may be a natural compound or compounds. The wet wipe of the present disclosure may be devoid of fragrance and/or perfume.

The lotions of the present disclosure may comprise natural plant extracts. The natural plant extracts may be selected from grape (Vitis vinifera) seed extract, Camellia sinensis (tea) leaf extract, Hamamelis virginiana leaf extract, Calendula officinalis flower extract, aloe vera, and combinations thereof.

The lotions of the present disclosure may comprise an odor reducing composition. The odor reducing composition may be a naturally derived odor reducing composition. The naturally derived odor reducing composition may manage odors inherent to the raw materials used in the manufacture of the wet wipe and/or manage odors associated with wet wipe (e.g., urine, feces, etc.). The naturally derived odor reducing composition may include plant extracts. Exemplary plant extracts may include magnolia extract and/or marigold extract, such as Extrapone® Marigold GW and Extrapone® Magnolia GW from Symrise. The naturally derived odor reducing composition may be substantially scent-free.

The lotions of the present disclosure may be incorporated onto a substrate at a load of about 200% to about 600%, from about 220% to about 500%, from about 240% to about 460%, or from about 240% to about 360% by weight of the nonwoven substrate, specifically reciting every 1% increment within these ranges and every range formed therein or thereby.

As discussed herein regarding the nonwoven substrates of the present disclosure, the lotion portion of the wet wipe products of the present disclosure may be non-uniformly distributed throughout the nonwoven substrate. Referring to Fig. 2, at least some of the plurality of elevated regions 200 may comprise a greater concentration of lotion than the non-elevated region 202 of the nonwoven substrate 100. The average concentration of lotion in the plurality of elevated regions 200 of a nonwoven substrate 100 of the present disclosure may be greater than the average concentration of lotion in the non-elevated region 202. Lotion distribution within a nonwoven substrate of the present disclosure may be visualized using short wave infrared camera analysis. This concentration of liquid in the elevated regions of the nonwoven substrate may provide an improved user cleaning experience, with increased cleaning liquid at the portions of the nonwoven substrate that are most in contact with the surface to be cleaned. It is believed that capillary action may draw lotion from the denser non-elevated region into the plurality of elevated regions of the nonwoven substrates of the present disclosure, thus providing a non-uniform distribution of lotion throughout the nonwoven substrate of the wet wipes.

*Packaging Materials*

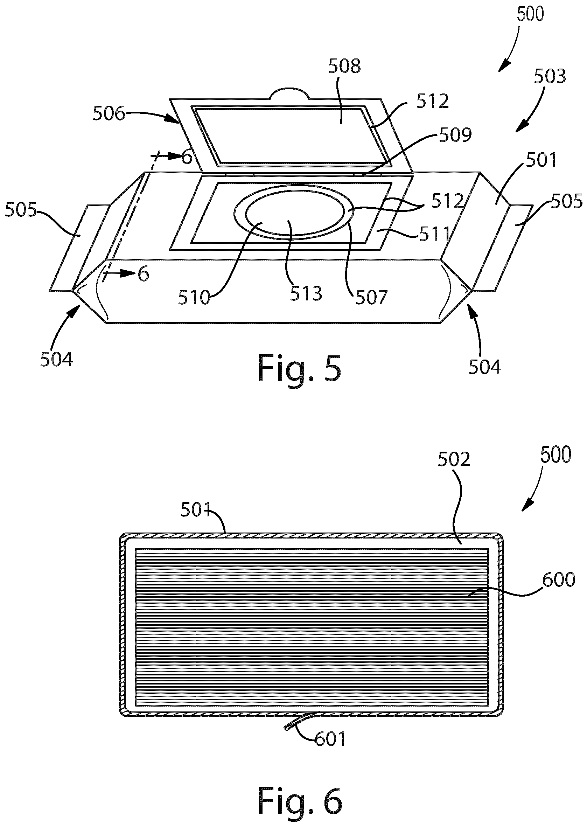

Figs. 5 and 6 depict a consumer product in the form of a packaged nonwoven substrate 500. The packaged nonwoven substrate 500 of the present disclosure comprises a packaging material 501 defining an interior package space 502. The packaging material 501 may comprise or consist of polystyrene, polypropylene, PET, POET, polyethylene, polyester, polyvinyl alcohol, or the like. The packaging material 501 may also be made of a mixture of the above materials. The packaging material 501 may form a package 503 in the form of a flexible flow wrap pouch, a rigid tub, or any other suitable configuration.

The packaging material 501 may be formed of a film that is flow-wrapped about a nonwoven substrate or stack of nonwoven substrates 600. The selected film material may be unwound from a stock roll and passed in a longitudinal/machine direction into a flow-wrap machine, along with individual nonwoven substrates or pluralities of nonwoven substrates 600. The flow-wrap machine may be configured so as to wrap the film stock longitudinally about each incoming nonwoven substrate or plurality of nonwoven substrates 600, join the film along its longitudinal edges to form a sealed fin seam 601 and a sleeve-like structure about the stack, tuck the film at the ends to form tucks 504, and then crimp, seal and cut the film between each nonwoven substrate or plurality of nonwoven substrates 600, forming individual packages 503 having end seams 505.

The package 503 may also be provided with an opening feature 506. The opening feature 506 may be a relatively rigid reclosable fitment. The fitment may have a ring portion 507 and a lid 508, connected to the ring portion 507 by a hinge 509. Ring portion 507 may have an opening 510 therethrough to provide access into the interior package space 502, surrounded by an escutcheon portion 511 that is adhered substantially along and about its perimeter to the packaging material 501 so as to provide a substantial moisture-proof seal between the escutcheon portion 511 and the packaging material 501. The lid 508 may have an open position and a closed position. One or both of lid 508 and ring portion 507 may be provided with lip, rim, groove, gasket, etc. cooperating sealing features 512 such that, when the lid is in the closed position, the cooperating sealing features 512 of the lid 508 and/or the ring portion 507 are in close proximity or effective contact with the other of lid 508 and/or ring portion 507 about the perimeter of the lid, so as to retard the passage of moisture between the lid 508 and the ring portion 507. One example may be a gasket (not specifically shown) formed of a material that is softer relative the material forming the fitment that may be provided about the opening 510 and disposed on either the lid 508 or ring portion 507, to improve the moisture passage retarding function. The fitment including the ring portion 507, hinge 509 and lid 508 may be formed of a polymer such as a polyolefin, for example, polyethylene. The fitment including the ring portion 507, hinge 509 and lid 508 may comprise a recycled material, such as a post-industrial recycled and/or post-consumer recycled polyolefin.

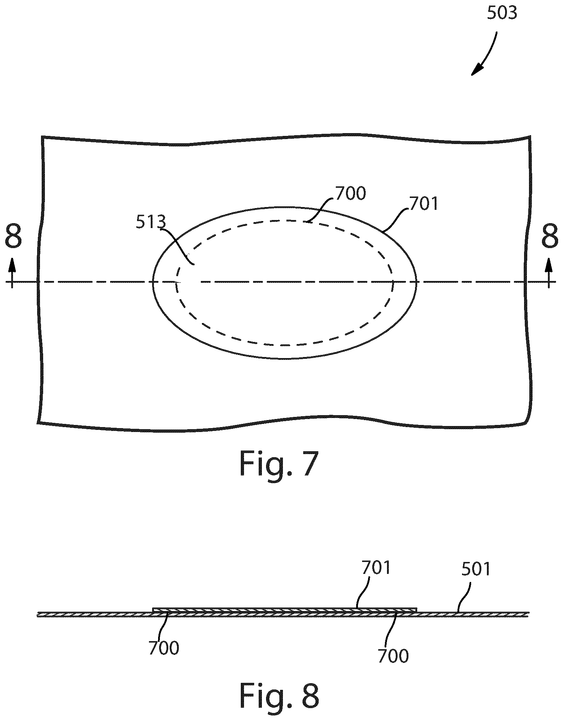

Referring to Figs. 5, 7, and 8, the fitment opening 510 may frame a package access opening 513 defined in the packaging material 501. Perforations 700 may outline a closed shape of the package access opening 513 through the packaging material 501. The perforations 700 are typically formed by a perforating die, and typically completely penetrate the packaging material. An opening feature flexible film 701 may be disposed on the packaging material 501 and over the package access opening 513, and may be of sufficient size to cover the entire package access opening 513 and/or all of the perforations 700 and thereby obstruct the passage of moisture therethrough. The opening feature flexible film may be in the form of an adhesive-backed film sticker, wherein the adhesive-backed side of the film sticker is removably adhered to a portion of the packaging material 501.

*Preservative Generation System*

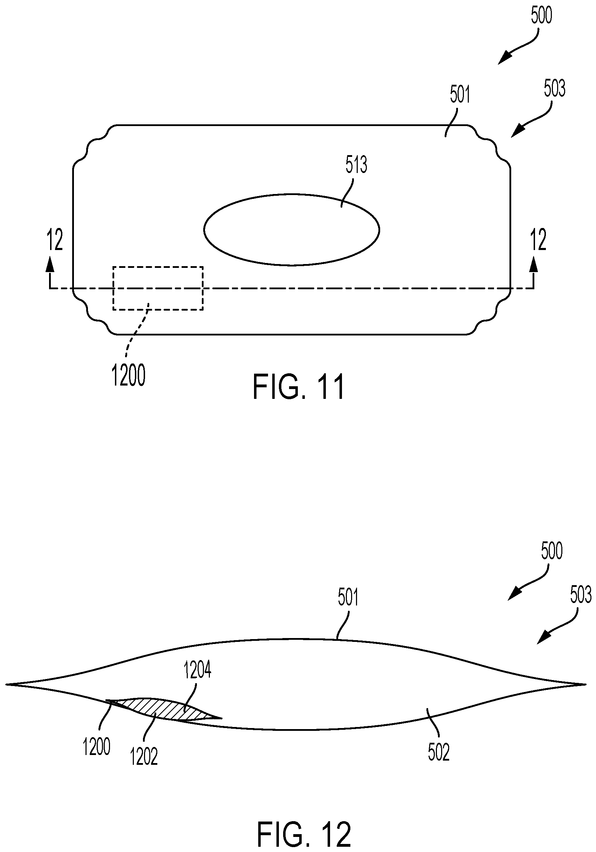

Packaged nonwoven substrates of the present disclosure may comprise a preservative generation system. Referring to Figs. 11 and 12, the preservative generation system may be fully enclosed by a packaging material 501 and disposed within the interior package space 502 of a closed package 503. The preservative generation system is configured to generate a preservative within the interior package space 502. The preservative may preserve, or assist in the preservation of, the nonwoven substrate, the lotion, and/or the consumer product as a whole. The preservative generation system may allow for the lotion to be free of preservatives. Where the consumer product comprises a preservative generation system, the lotion may consist of water. Exemplary preservative generation systems are described in U.S. Patent No. 6,607,696; U.S. Patent No. 6,602,466; and U.S. Patent No. 7,922,984, each of which is herein incorporated by reference.

As shown in Figs. 11, 12, and 13, the preservative generation system may comprise a first pouch 1200. The first pouch 1200 may define a closed receptacle around a reactant 1202. The first pouch 1200 may comprise a first semi-permeable material. The first semi-permeable material may be designed and/or configured to allow entry of water and/or water vapor into the first pouch 1200. The first semi-permeable material may be designed and/or configured to allow egress of a preservative, for example a preservative gas. The first semi-permeable material may comprise or be formed of perforated films, non-perforated films, and/or selective transmission films, and combinations thereof.

The first pouch 1200 comprising or formed of a perforated film may have a water vapor transmission rate (WVTR) between about 50 $g/m^2/24$ hours and about 1,000 $g/m^2/24$ hours, between about 200 $g/m^2/24$ hours and about 800 $g/m^2/24$ hours, or between about 400 $g/m^2/24$ hours and about 700 $g/m^2/24$ hours, specifically reciting every 1 $g/m^2/24$ hours increment within these ranges and any ranges formed therein or thereby. The perforated film may be hydrophobic. Perforated films suitable for the construction of the envelope may include, but are not limited to, polymeric material, e.g., Cryovac® perforated films available from SEALED AIR CORPORATION (Duncan, S.C.). One such film is a hydrophobic polypropylene copolymer film sold under the designation SM700 by SEALED AIR CORPORATION and has 330 holes per square inch having a diameter of 0.4 mm, a 6.4% perforated area, a thickness of about 20 microns, and a water vapor transmission rate of 700 $g/m^2/24$ hrs. Another suitable film is a hydrophobic polypropylene copolymer film sold under the designation SM60 by SEALED AIR CORPORATION and has 8 holes per square inch having a diameter of 0.4 mm, a 0.2% perforated area and a water vapor transmission rate of 65 $g/m^2/24$ hrs.

The first pouch 1200 may comprise or be formed of hydrophobic, liquid water permeable material, such as polyethylene or polypropylene. These materials preferably are between about 1 mil (0.0254 mm) and about 10 mils (0.254 mm) thick with a water intrusion pressure of about 30 millibars or 30 millibars or less. Hydrophobic materials may include, but are not limited to, non-woven polyethylene such as the TYVEK® non-woven polyethylenes from DUPONT Company (Wilmington, Del.), e.g., the TYVEK® 1025D non-woven polyethylene which has an intrusion pressure of less than 30 millibars.

The first pouch 1200 may comprise, or be formed from, a hydrophilic membrane having a pore size between about 0.01 $\mu$m and about 50 $\mu$m, between about 0.05 $\mu$m and 40 $\mu$m, or between about 0.1 $\mu$m and about 30 $\mu$m. The membrane may be the microporous ultra-high density polyethylene membrane sold under the trade designation MPLC from MILLIPORE (Bedford, Mass.), and the microporous Nylon 6,6 membrane sold under the designation 045ZY by CUNO Incorporated (Meriden, Conn.).

Selective transmission films are films that are neither perforated nor porous, but instead transfer gases through the polymer structure of the film. Selective transmission films are multilayered or mixed polymer materials, where the layers and the polymers are chosen for controlled transmission of gases such as carbon dioxide, chlorine dioxide, and/or oxygen. Selective transmission films may be beneficial in dry applications because they allow gas to diffuse out of the first pouch, while retaining an initiating agent and reactant. Moreover, the selective transmission film may increase the stability of the preservative generation system prior to its use because it does not easily allow ambient water and/or water vapor to diffuse into the first pouch 1200, which could prematurely initiate reactants.

The first pouch 1200 may comprise a reactant 1202 disposed within an interior 1204 of the first pouch 1200, wherein the reactant is configured to generate a preservative upon exposure to water and/or water vapor. The reactant 1202 may be a compound or a mixture of compounds configured to generate the preservative. The preservative may be in a gas phase. Generation of a gas, *e.g.,* by acid activation, is well known in the art. For example, chlorine dioxide ($ClO_2$) may be generated from sodium chlorite and an acid, such as citric acid, in the presence of moisture. Alternatively, chlorine dioxide can be produced by the reduction of a chlorate, e.g., sodium chlorate or potassium chlorate, in the presence of an acid, e.g., oxalic acid. The reactant may comprise at least one of: sodium chlorite, potassium chlorite, citric acid, and combinations thereof.

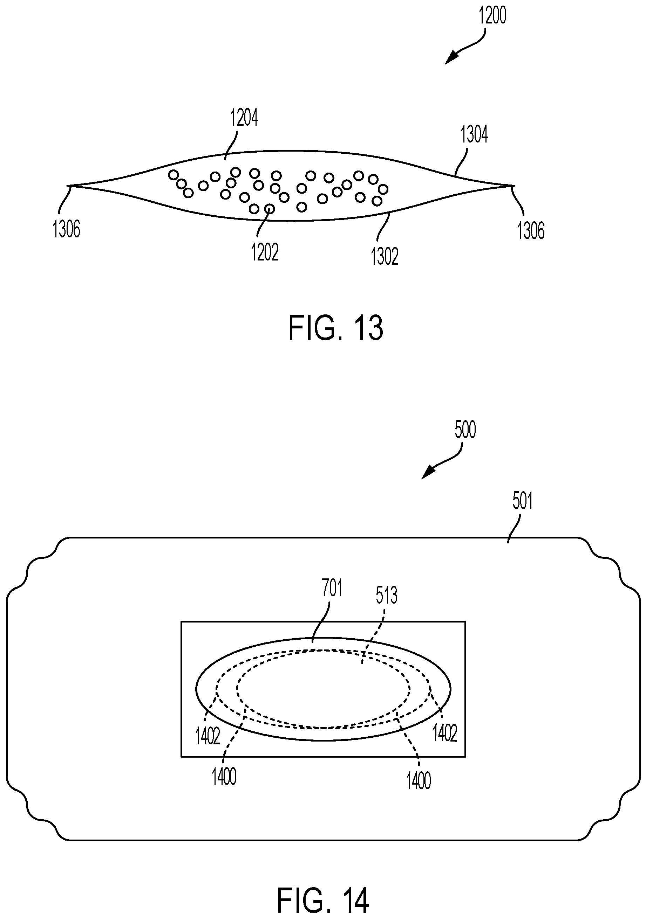

Referring to Fig. 13, the first pouch 1200 may comprise a first side 1302 and a second side 1304. The first side 1302 and the second side 1304 may each be formed by separate pieces of material which are attached at pouch seams 1306. The first side 1302 of the first pouch 1200 may be joined and/or bonded to the packaging material 501. The first side 1302 of the first pouch 1200 may be adhesively joined to the packaging material 501. At least a portion of the second side 1304 of the first pouch 1200 may be free of attachment to the packaging material 501. The second side 1304 of the first pouch 1200 may comprise or be formed from the semi-permeable material. The first pouch 1200 may be sealed such that the reactant 1202 is disposed only in the interior 1204 of the first pouch 1200. The first pouch 1200 may be configured to allow the ingress of water and/or water vapor. The first pouch 1200 may be configured to allow the egress of the preservative, while maintaining the reactant 1202 within the interior 1204 of the first pouch 1200. The preservative may be chlorine dioxide gas.

Individual packages of the packaged nonwoven substrates of the present disclosure may be formed by sealing the nonwoven substrate, a lotion, and a preservative generation system within the interior package space formed by the packaging material. The preservative generation system, and, more specifically, the first pouch, may come into contact with water and/or water vapor only upon assembly of the packaged consumer product, where the preservative generation system comes into contact with the lotion portion of the packaged consumer product. The water and/or water vapor from the lotion may cross the semi-permeable material of the first pouch during or shortly after assembly of the packaged consumer product and may come into contact with the reactant, initiating a preservative generation reaction with the reactant. The reaction may form a preservative or preservatives. The preservative or preservatives may be in the form of a gas. The preservative or preservatives may permeate the semi-permeable material and enter the interior package space. The packaging material 501 defining and surrounding the interior package space 502 may be impermeable to the preservative(s) and may hold the preservative(s) within the interior package space until the package is opened.

The preservative may be contained within the interior package space 502 and may be present in a sufficient amount to reduce, inhibit, and/or eliminate bacteria, yeast, and/or mold in and/or on consumer product elements within the interior package space 502 while the package is sealed (prior to opening - by, for example, removal of the opening feature flexible film 701). The preservative may be chlorine dioxide. The interior package space 502 of a sealed package 503 may comprise a gaseous phase. The gaseous phase may comprise between about 0.25 ppm and about 5 ppm chlorine dioxide, between about 0.3 ppm and about 4.5 ppm chlorine dioxide, between about 0.35 ppm and about 3.75 ppm chlorine dioxide, or between about 0.45 and about 4.25 ppm chlorine dioxide, specifically reciting ever 0.01 ppm increment within these ranges and any ranges formed therein or thereby.

The inventors have found that the level of chlorine dioxide generated by the preservative generation system is capable of reducing, inhibiting, and/or eliminating bacteria, mold, and/or yeast while leaving very little residual chlorate/chlorite species on and/or in either the nonwoven substrate or the lotion. When tested upon opening of the packaged consumer product of the present disclosure, the nonwoven substrates may comprise less than 1000 $\mu$g/ml chlorate and/or chlorite species, less than 800 $\mu$g/ml chlorate and/or chlorite species, less than 650 $\mu$g/ml chlorate and/or chlorite species, less than 500 $\mu$g/ml chlorate and/or chlorite species, or less than 375 $\mu$g/ml chlorate and/or chlorite species. When tested upon opening of the packaged consumer product of the present disclosure, the lotion may comprise less than 1000 $\mu$g/ml chlorate and/or chlorite species, less than 800 $\mu$g/ml chlorate and/or chlorite species, less than 650 $\mu$g/ml chlorate and/or chlorite species, less than 500 $\mu$g/ml chlorate and/or chlorite species, or less than 375 $\mu$g/ml chlorate and/or chlorite species.

The preservative generation system of the present disclosure may comprise a second pouch disposed in the interior package space. The second pouch may define a closed receptacle around a second reactant. The second pouch may comprise a second semi-permeable material. The second semi-permeable material may be designed and/or configured to allow entry of water and/or water vapor into the second pouch. The second semi-permeable material may be designed and/or configured to allow egress of a preservative, for example a preservative gas. The second semi-permeable material may comprise or be formed of perforated films, non-perforated films, and/or selective transmission films, and combinations thereof.

The second semi-permeable material of the second pouch may be configured to be shielded from contact with water, water vapor, and/or the lotion when the outer package is in a first, unopened configuration - meaning post-manufacture but pre-consumer use. When the outer package is in a second, opened configuration, the second semi-permeable material of the second pouch may be configured to be in contact with water, water vapor, and/or the lotion.

Referring to Fig. 14, the second pouch 1400 may be disposed around at least a portion of the package access opening 513 such that at least a portion of the second semi-permeable material 1402 may be free from attachment to the packaging material 501. When the outer package is in the first, unopened configuration at least a portion of the second semi-permeable material 1402 may be joined to the opening feature flexible film 701. The portion of the second semi-permeable material 1402 that is free from attachment to the packaging material 501 may be the portion attached to the opening feature flexible film 701, such that no portion of the second semi-permeable material 1402 is exposed to a source of moisture, such as a lotion. Thus, the second semi-permeable material 1402 is shielded from exposure to water and/or water vapor - such as that from the lotion. Such a configuration may prevent generation of a preservative from the second pouch while the package is in a sealed configuration.

The first pouch and/or the second pouch may be sealed, such as at pouch seams 1306, in order that the reactant remains within the interior space of the respective pouch and is substantially or completely prevented from entering into the interior package space.

In a form, the first pouch and/or the second pouch may be formed by a portion of the packaging material forming a first side of the pouch, and a semi-permeable material forming a portion of a second side of the pouch. Reactant may be disposed within an interior space between the first side and second side. The reactant may be printed, slot-coated, or otherwise deposited on the packaging material before the semi-permeable material is joined to the packaging material to form the first and/or second pouch.

## Methods and Equipment for Manufacturing Nonwoven Substrates

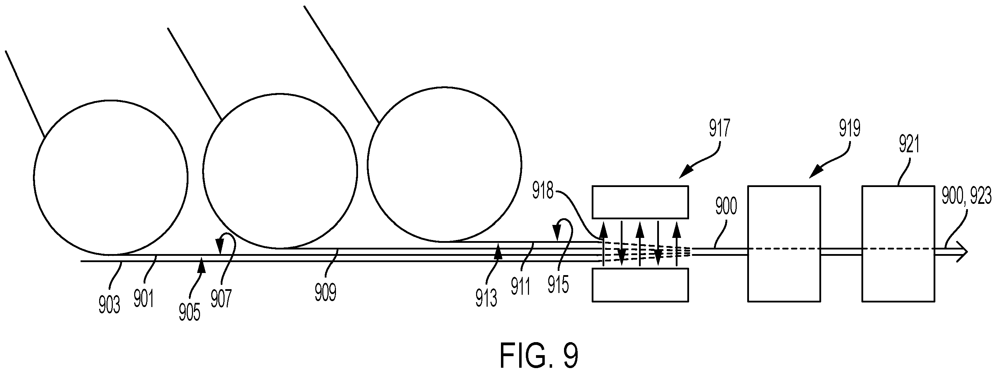

The present disclosure is directed, in part, to methods and equipment for making nonwoven substrates on manufacturing lines. Referring to Fig. 9, the nonwoven substrate 900 may be formed by depositing a first plurality of fibers 901 on a forming belt 903. A first outer surface 905 of the first plurality of fibers 901 may be in face-to-face relationship with the forming belt 903, and a first interior surface 907 of the first plurality of fibers 901 may be opposite the first outer surface 905 and may face away from the forming belt. The first plurality of fibers 901 may be deposited as a preformed batt of material, for example being unwound from a roll of preformed material as shown in Fig. 9. The first plurality of fibers 901 may be deposited by any method known in the art, including air-laying, wet-laying, or melt spinning.

As depicted in Fig. 9, a separate plurality of fibers 909 may be deposited on the first plurality of fibers 901, such that one side of the separate plurality of fibers 909 is in face-to-face relationship with the first interior surface of the first plurality of fibers 901. The separate plurality of fibers 909 may be the third material layer 114 and/or the third plurality of fibers 118 as described herein. The third plurality of fibers 909 may be deposited as a preformed batt of material, such as, for example, a preformed tissue layer. The third plurality of fibers 909 may comprise individual fibers that are bonded to one another, such that the third plurality of fibers 909 forms a cohesive batt of material. At least a portion of the individual fibers of the third plurality of fibers 909 may be covalently bonded to one another through the use of a binder agent, a wet-strength agent, or the like. At least a portion of the individual fibers of the third plurality of fibers 909 may be hydrogen bonded to one another without the use of a binder agent, a wet-strength agent, or the like.

The method of forming the nonwoven substrates of the present disclosure further comprises depositing a second separate plurality of fibers 911 on the third plurality of fibers 909, such that a second interior surface 913 of the second plurality of fibers 911 is disposed in face-to-face relationship with the third plurality of fibers 909, and a second outer surface 915 of the second plurality of fibers 911 faces away from the third plurality of fibers 909. The second plurality of fibers 911 may be deposited as a preformed batt of material, for example being unwound from a roll of preformed material as shown in Fig. 9. The second plurality of fibers 911 may be deposited by any method known in the art, including air-laying, wet-laying, or melt spinning. The second plurality of fibers 911 may correspond to the second material layer 108 and/or the second plurality of fibers 110 of the nonwoven substrates of the present disclosure discussed herein.

As shown in Fig. 9, the method of forming the nonwoven substrates of the present disclosure further comprises bonding the first plurality of fibers 901, the second plurality of fibers 909, and the third plurality of fibers 911 to form the nonwoven substrate 900. The bonding step may include thermal bonding techniques, such as embossing, air-through bonding, calendaring, and the like. The bonding step may comprise hydraulically entangling the first plurality of fibers 901, the second plurality of fibers 909, and the third plurality of fibers 911 together. The three pluralities of fibers 901, 909, 911, may be subjected to a first hydroentangling process by applying hydraulic energy to the first outer surface 905 and/or a second outer surface 915. The three pluralities of fibers 901, 909, 911, may be subjected to a second hydroentangling process by applying hydraulic energy to the opposite outer surface as in the first hydroentangling process. By way of example, the first hydroentangling process may apply hydraulic energy to the first outer surface 905, while the second hydroentangling process may apply hydraulic energy to the second outer surface 915, or the first hydroentangling process may apply hydraulic energy to the second outer surface 915, while the second hydroentangling process may apply hydraulic energy to the first outer surface 905. The hydroentangling processes may be applied concurrently to both the first outer surface 905 and the second outer surface 915, as shown in Fig. 9, or successively.

A hydroentangling device 917 may be employed to bond the first plurality of fibers 901, the second plurality of fibers 909, and the third plurality of fibers 911 to form the nonwoven substrate 900. The hydroentangling device 917 may be configured to pass a fluid 918 through an orifice or a plurality of orifices under high pressure. The hydroentangling fluid 918 is directed at one or both outer surfaces of the pluralities of fibers 901, 909, 911 on the forming belt. The hydroentangling fluid 918 impacts the pluralities of fibers 901, 909, 911, causing at least some individual fibers from each plurality to entangle and form a cohesive nonwoven substrate 900. The hydroentangling process may also break some, or all, the covalent and/or hydrogen bonds between the individual fibers of the third plurality of fibers, resulting in the third plurality of fibers to be at least partially, or fully, individualized. The first plurality of fibers 901 may form the first material layer 102, as depicted in Fig. 1, or the first plurality of fibers 901 may form the second material layer 108. Likewise, the second plurality of fibers 909 may form the second material layer 108, as depicted in Fig. 1, or the second plurality of fibers 909 may form the first material layer 102.

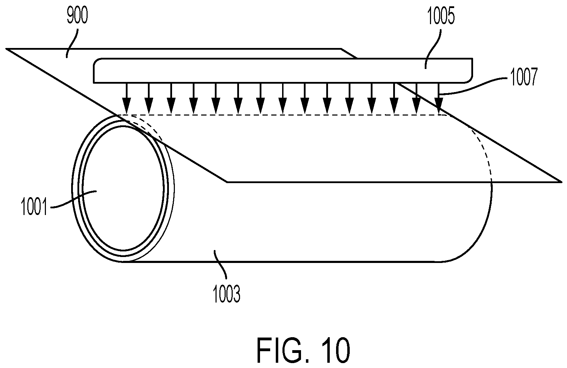

Referring again to Fig. 9, the method of forming the nonwoven substrates of the present disclosure may further comprise patterning at least one outer surface of the nonwoven substrate. The nonwoven substrate 900 may pass through a patterning device 919 configured to impart a three-dimensional pattern into an outer surface of the nonwoven substrate 900. Patterning may comprise hydraulicly patterning, or hydromolding, the nonwoven substrate. As shown in Fig. 10, the patterning device 919 may comprise a support 1001 having a design engraved or recessed into the support surface 1003. The support may be formed from a screen-like material, may comprise apertures therein, or may otherwise allow for fluid to drain away from the support surface. As shown in Fig. 10, the support 1001 may be in the form of a roller. In other forms, the support may be a belt. The support 1001 is hollow, allowing liquid to drain away. The patterning device 919 may further comprise a hydraulic needling manifold 1005. The hydraulic needling manifold 1005 may be positioned above the support 1001 and may produce a plurality of fine fluid jets 1007. The nonwoven substrate 900 passes between the support 1001 and the hydraulic needling manifold 1005, wherein the fluid jets 1007 impact the nonwoven substrate 900, causing the formation of a three-dimensional structure in at least one outer surface of the nonwoven substrate 900. Applying the patterning step to the nonwoven substrate 900 may result in the creation of a patterned nonwoven substrate 923.

The three-dimensional structure formed by the patterning step may comprise a plurality of elevated regions 200 and a non-elevated region 202 disposed between the plurality of elevated regions 200, as shown in Figs. 2-4 and described herein. It is believed that, due to the impact of the fluid jets on the nonwoven substrate 100, 900, at least a portion of the third plurality of fibers 116, 911 - which are sandwiched between the first material layer 102 and the second material layer 108 prior to the patterning step - may be pushed through to at least one of the first material layer 102 and second material layer 108, and may ultimately be disposed on the first outer surface 106 and/or the second outer surface 112 in at least some of the plurality of elevated regions 200.

The rearrangement of fibers within the nonwoven substrate is believed to be due, in part, to the impact of the liquid jets of the bonding and/or patterning step. It is believed that, as the fluid jets impact the nonwoven substrate, the mechanical force of the liquid jets is sufficient to break some or all of the bonds holding the fibers of the third plurality of fibers together, causing at least some, or all, of the fibers of the third plurality of fibers to become singularized and to intertwine with some of the fibers of an adjacent material layer. Where the third material layer is introduced into the manufacturing process in the form of a tissue layer, for example, the mechanical energy applied during the bonding and/or patterning step may break some of, or all, the covalent and/or hydrogen bonds between the pulp fibers and may allow a portion of the fibers of the third plurality of fibers to protrude into an adjacent layer and to reach and reside on an outer surface of the nonwoven substrate. In areas where the fluid jets do not impact the nonwoven substrate, the regions may remain non-elevated and a portion of the third plurality of fibers in the non-elevated region(s) may remain bonded to one another.

The nonwoven substrate 900 may be dried using conventional processes. As shown in Fig. 9, the nonwoven substrate may pass through a drying apparatus 921 after being patterned. Alternatively, or in addition, the nonwoven substrate 900 may be passed through a drying apparatus after the bonding step, especially when the bonding step comprises hydroentangling.

*Method Combinations:*

1. A method of manufacturing a nonwoven substrate, the method comprising:

depositing a first plurality of fibers on a forming belt, the first plurality of fibers forming a first material layer comprising a first outer surface in face-to-face relationship with the forming belt and a first interior surface opposite the first outer surface;
depositing a third plurality of fibers on the first material layer in a face-to-face relationship with the first interior surface of the first material layer and forming a third material layer, wherein the third plurality of fibers comprises bonded fibers forming a tissue;
depositing a second plurality of fibers on the third material layer, the second plurality of fibers forming a second material layer comprising a second interior surface disposed in face-to-face relationship with the third material layer and a second outer surface opposite the second interior layer; and
bonding the first material layer, third material layer, and second material layer to form the nonwoven substrate.

2. A method of manufacturing a nonwoven substrate, the method comprising:

depositing a second plurality of fibers on a forming belt, the second plurality of fibers forming a second material layer comprising a second outer surface in face-to-face relationship with the forming belt and a second interior surface opposite the second outer surface;
depositing a third plurality of fibers on the second material layer in a face-to-face relationship with the second interior surface of the second material layer and forming a third material layer, wherein the third plurality of fibers comprises bonded fibers forming a tissue;
depositing a first plurality of fibers on the third material layer, the first plurality of fibers forming a first material layer comprising a first interior surface disposed in face-to-face relationship with the third material layer and a first outer surface opposite the first interior layer; and
bonding the first material layer, third material layer, and second material layer to form a nonwoven substrate.

3. The method of any of paragraphs 1 and 2, wherein the first plurality of fibers and/or the second plurality of fibers is deposited as a preformed batt of material.

4. The method of any of the preceding paragraphs, wherein the third plurality of fibers is deposited as a preformed tissue sheet.

5. The method of paragraph 4, wherein the third plurality of fibers are hydrogen bonded to one another.

6. The method of any of paragraphs 4 or 5, wherein the third material layer is free of binder agents and wet strength agents.

7. The method of any of the preceding paragraphs, wherein the bonding step comprises hydroentangling the first material layer, the second material layer, and the third material layer using a high pressure fluid jet.

8. The method of an of the preceding paragraphs further comprising the step of drying the nonwoven substrate.

9. The method of any of the preceding paragraphs, wherein the first plurality of fibers and/or the second plurality of fibers comprises carded fibers having an average fiber length of between about 20 mm and about 80 mm, between about 25 mm and about 75 mm, between about 30 mm and about 70 mm, or between about 35 mm and about 65 mm.

10. The method of paragraph 9, wherein the first plurality of fibers and/or the second plurality of fibers consists of carded fibers having an average fiber length of between about 20 mm and about 80 mm, between about 25 mm and about 75 mm, between about 30 mm and about 70 mm, or between about 35 mm and about 65 mm.

11. The method of any of paragraphs 9 or 10, wherein the carded fibers comprise lyocell.

12. The method of any of paragraphs 9-11, wherein the carded fibers consist of lyocell.

13. The method of any of the preceding paragraphs, wherein the third plurality of fibers comprise natural fibers.

14. The method of any of the preceding paragraphs, wherein the third plurality of fibers consists of natural fibers.

15. The method of any of paragraphs 13 or 14, wherein the natural fibers comprise pulp fibers.

16. The method of any of paragraphs 13-15, wherein the natural fibers consist of pulp fibers.

17. The method of any of the preceding paragraphs, further comprising patterning the nonwoven substrate.

18. The method of paragraph 17, wherein patterning the nonwoven substrate comprises hydromolding the nonwoven substrate.

19. The method of paragraphs 17-18, wherein the patterning step forms a three-dimensional structure onto the first outer surface and/or the second outer surface of the nonwoven substrate, forming a patterned nonwoven substrate.

20. The method of paragraph 19, wherein the three-dimensional structure comprises a plurality of elevated regions and a non-elevated region disposed between the plurality of elevated regions.

21. The method of any of paragraphs 19-20, wherein fibers of the third plurality of fibers are disposed on the first outer surface and/or the second outer surface in at least some of the plurality of elevated regions.

22. The method of any of paragraphs 19-21, wherein at least some of the fibers of the third plurality of fibers are intertwined with some of the fibers of the first plurality of fibers and/or the second plurality of fibers in at least some of the elevated regions.

23. The method of any of paragraphs 19-22, wherein the third plurality of fibers are disposed substantially between the first material layer and the second material layer in non-elevated regions.

24. The method of any of the preceding paragraphs, wherein the fibers of the third plurality of fibers are not bonded to one another in the nonwoven substrate.

Absorbent Articles

The nonwoven substrates of the present disclosure may be used as components and or layers of components of absorbent articles.

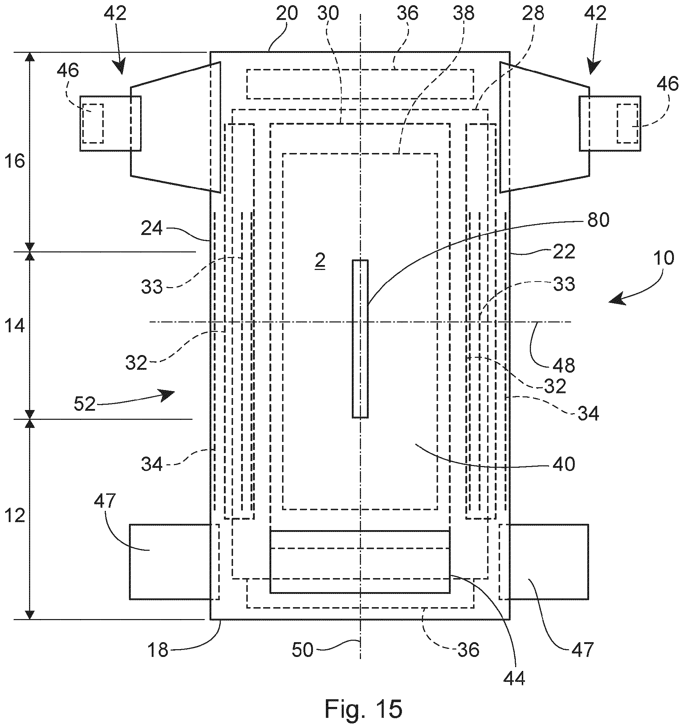

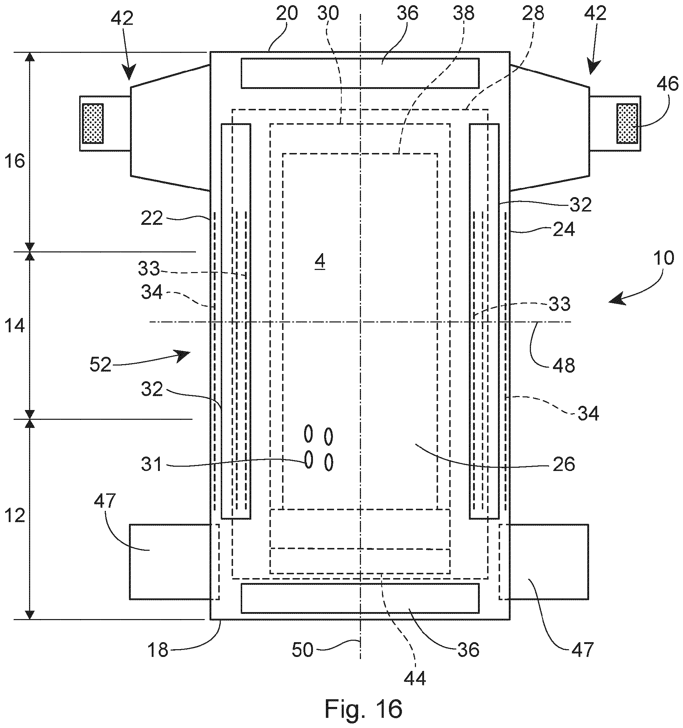

An example absorbent article 10 according to the present disclosure, shown in the form of a taped diaper, is represented in Figs. 15-16. Fig. 15 is a plan view of the example absorbent article 10, garment-facing surface 2 facing the viewer in a flat, laid-out state (i.e., no elastic contraction). Fig. 16 is a plan view of the example absorbent article 10 of Fig. 15, wearer-facing surface 4 facing the viewer in a flat, laid-out state. The absorbent article 10 of Figs. 15-16 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 may comprise a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137.

*Topsheet*

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet 26 may comprise or be formed entirely from the nonwoven substrates of the present disclosure. The topsheet may have one or more layers. The topsheet may be apertured (Fig. 16, element 31), may have any suitable three-dimensional features, and/or may have a plurality of embossments (e.g., a bond pattern). The topsheet may comprise a variable basis weight nonwoven material, such as those described in U.S. Pat. Appl. No. 2017/0191198. The topsheet may be apertured by overbonding a material and then rupturing the overbonds through ring rolling, such as disclosed in U.S. Patent No. 5,628,097, to Benson et al., issued on May 13, 1997, and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet. The topsheet may comprise a first material layer comprising a first plurality of fibers, a second material layer comprising a second plurality of fibers, and a third material layer comprising a third plurality of fibers. The topsheet may have a first outer surface and a second outer surface opposite the first outer surface. A plurality of elevated regions may be disposed on the first outer surface and a plurality of non-elevated regions are disposed on the first outer surface and between the plurality of elevated regions. Fibers of the third plurality of fibers may reside on the first outer surface in at least some of the plurality of elevated regions.

*Backsheet*

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials, such as films, which permit vapors to escape from the absorbent article, while still preventing, or at least inhibiting, bodily exudates from passing through the backsheet. The backsheet 28 may be coterminous with the outer cover material 40.

*Outer Cover Material*

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material or a variable basis weight nonwoven material. The outer cover material 40 may comprise or may be formed entirely from the nonwoven substrates of the present disclosure. The outer cover material 40 may comprise a first material layer comprising a first plurality of fibers, a second material layer comprising a second plurality of fibers, and a third material layer comprising a third plurality of fibers. The outer cover material 40 may have a first outer surface and a second outer surface opposite the first outer surface. A plurality of elevated regions may be disposed on the first outer surface and a plurality of non-elevated regions are disposed on the first outer surface and between the plurality of elevated regions. Fibers of the third plurality of fibers may reside on the first outer surface in at least some of the plurality of elevated regions.

*Absorbent Core*

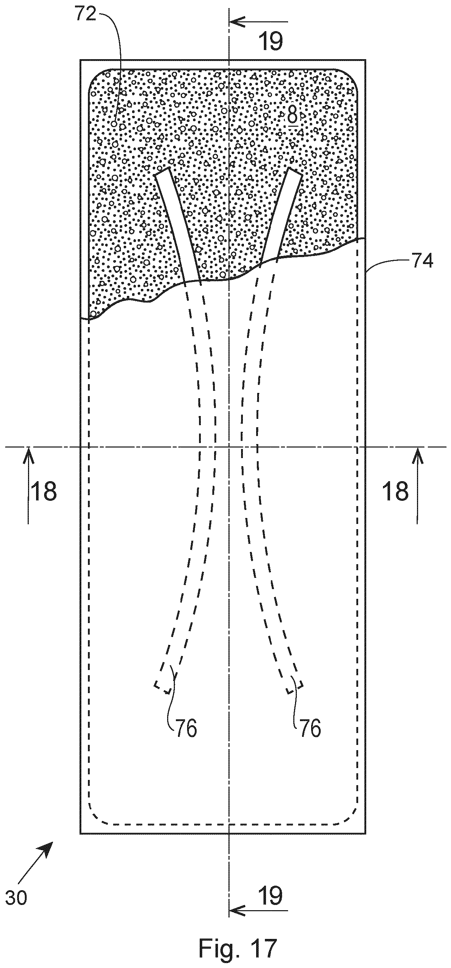

As used herein, the term "absorbent core" refers to a component of the absorbent article 10 for absorbing and containing liquid such as urine received by the absorbent article. The absorbent core thus typically has a high absorbent capacity. An example absorbent core 30 is schematically shown in Figs. 17-19. The absorbent core comprises an absorbent material 72. The absorbent core 30 typically comprises a core wrap 74 that encloses or sandwiches the absorbent material. The core wrap may be a single material that is folded and attached to itself, or it may comprise a separate top layer and bottom layer that may be bonded, adhesively joined, or otherwise joined together. The top and bottom layers of the core wrap may be the same or different. The core wrap 74 may comprise or may be formed entirely from the nonwoven substrates of the present disclosure. The core wrap 74 may comprise a first material layer comprising a first plurality of fibers, a second material layer comprising a second plurality of fibers, and a third material layer comprising a third plurality of fibers. The core wrap may have a first outer surface and a second outer surface opposite the first outer surface. A plurality of elevated regions may be disposed on the first outer surface and a plurality of non-elevated regions are disposed on the first outer surface and between the plurality of elevated regions. Fibers of the third plurality of fibers may reside on the first outer surface in at least some of the plurality of elevated regions.

The absorbent material typically comprises superabsorbent particles which are optionally mixed with cellulose fibers. As used herein, "absorbent core" does not include any acquisition-distribution systems, topsheet, or backsheet of the absorbent article.

The example absorbent core 30 shown in isolation in Figs. 17-19 is in the dry state (before use). The absorbent core may typically have a generally rectangular shape as defined by its longitudinal edges and transversal front edge and back edge or may have other shapes.

Absorbent material 72 may be deposited as an absorbent layer having a generally rectangular outline, as represented in Fig. 17. A wide variety of absorbent cores may also be used. The absorbent material 72 layer may also have a non-rectangular perimeter ("shaped" core), in particular, the absorbent material 72 may define a tapering along its width towards the central region of the core (or "dog-bone" shape). In this way, the absorbent material deposition area may have a relatively narrow width in an area of the core intended to be placed in the crotch region of the absorbent article. This may provide for example better wearing comfort. Other shapes can also be used such as a "T" or "Y" or "hourglass" for the area of the absorbent material.

The absorbent material 72 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam. The absorbent material may also be a coform material comprising a nonwoven web containing a mixture of staple fibers and an absorbent material including pulp fibers, the staple fibers being present in the nonwoven web in an amount of from about 5wt. % to about 50wt.%, the pulp fibers being present in the nonwoven web in an amount of from about 50wt.% to about 95wt.%, wherein the staple fibers have an average length of from about 5 mm to about 50 mm, and wherein the staple fibers and pulp fibers are thermally bonded or hydraulically entangled to form the nonwoven web (as exemplary disclosed in WO2023/022979).

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from about 20 g/g to about 40 g/g. "Superabsorbent polymer particles", as used herein, refers to a superabsorbent polymer material which is in particulate form so as to be flowable in the dry state.

Various absorbent core designs comprising high amounts of SAP have been proposed in the past, see for example in U.S. Pat. No. 5,599,335 (Goldman), EP1,447,066 (Busam), WO95/11652 (Tanzer), U.S. Pat. Appl. Pub. No. 2008/0312622A1 (Hundorf), WO2012/052172 (Van Malderen). In particular, the SAP printing technology as disclosed in U.S. Pat. No. 7,838,722 (Blessing), U.S. Pat. No. 9,072,634 and U.S. Pat. No. 8,206,533 (both to Hundorf et al.) may be used. The present disclosure however is not limited to a particular type of absorbent core. The absorbent core may also comprise one or more glues such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate, such as a carded nonwoven layer, having a porous structure into which SAP particles have been deposited, may also be used in present disclosure.

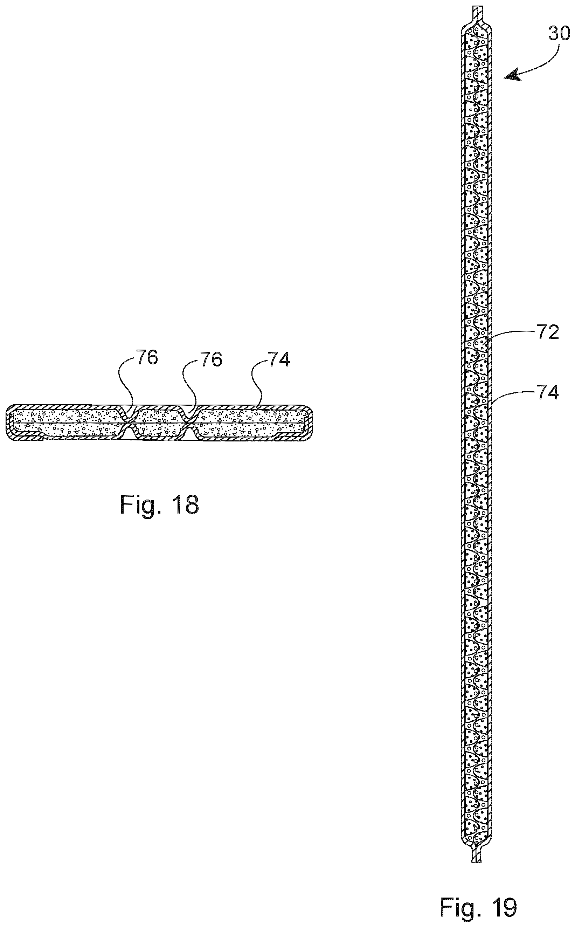

The absorbent material may be deposited as a continuous layer within the core wrap. The absorbent material may also be present discontinuously, for example, as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern, wherein the resulting layer is substantially continuously distributed across the absorbent particulate polymer material area, as illustrated in Figs. 18-19. As for example taught in U.S. Pat. Appl. Pub. No. 2008/0312622 A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally (or otherwise) extending channels 76, which are areas of the absorbent layer substantially free of absorbent material within the absorbent material layer. The top side of the core wrap may be advantageously bonded to the bottom side of the core by adhesive, mechanical or ultra-sonic bonding through these material-free areas. Example disclosures of such channels in an airfelt-free core can be found in U.S. Pat.

No. 9,789,011. One or more channels may also be formed in absorbent cores comprising a mix of cellulose fibers and SAP particles. These channels may embody any suitable shapes and any suitable number of channels may be provided. In other instances, the absorbent core may be embossed to create the impression of channels, with absorbent material remaining within the channels. The absorbent core in Figs. 17-19 is merely an example absorbent core. Many other absorbent cores with or without channels are also within the scope of the present disclosure.

*Acquisition-Distribution Materials*

Referring to Figs. 1 and 2, one or more acquisition-distribution materials 38 may be present at least partially intermediate the topsheet 26 and the absorbent core 30. The acquisition-distribution materials 38 are typically hydrophilic materials that provide significant wicking of bodily exudates. These materials may dewater the topsheet 26 and quickly move bodily exudates into the absorbent core 30. The acquisition-distribution materials 38 may comprise one or more nonwoven materials, foams, formed films, apertured formed films, cellulosic materials, cross-linked cellulosic materials, air laid cellulosic nonwoven materials, spunlace materials, or combinations thereof, for example. The acquisition-distribution material may be rectangular or may be shaped, such as hourglass shaped. Typically, an acquisition-distribution material 38 may have a width and length that are smaller than the width and length of the topsheet 26. The acquisition-distribution material may be a secondary topsheet in the feminine pad context. The acquisition-distribution materials may have one or more channels as described above with reference to the absorbent core 30 (including the embossed version). The channels in the acquisition-distribution material may align or not align with channels in the absorbent core 30. In an example, a first acquisition-distribution material may comprise a nonwoven material and as second acquisition-distribution material may comprise a cross-linked cellulosic material. The acquisition-distribution material may comprise or may be formed entirely from the nonwoven substrates of the present disclosure. The acquisition-distribution materials 38 may comprise a first material layer comprising a first plurality of fibers, a second material layer comprising a second plurality of fibers, and a third material layer comprising a third plurality of fibers. The acquisition-distribution materials 38 may have a first outer surface and a second outer surface opposite the first outer surface. A plurality of elevated regions may be disposed on the first outer surface and a plurality of non-elevated regions are disposed on the first outer surface and between the plurality of elevated regions. Fibers of the third plurality of fibers may reside on the first outer surface in at least some of the plurality of elevated regions.

Test Methods

All test methods are carried out in an environment 23 ± 2 °C and 50 ± 5% relative humidity environment, unless otherwise specified.

*Lotion Expression Method*

Lotion is expressed from wet wipes for further analysis using the Lotion Expression Method. In this method, lotion is extracted from one or more like packages of lotioned wipes that have been wetted in a sealed package for at least 28 days at 40°C and 75% relative humidity in order to collect a desired quantity of lotion for further analysis.

One corner is cut from one sealed wipes package to create a small (approximately 0.25-inch in diameter) hole from which liquid can escape. The package is then positioned between the platens of a uniaxial press with the corner containing the hole for liquid escape hanging outside the edge of the platens such that lotion can be collected in a suitable container as pressure is applied. Pressure is then increasingly applied to the package of wipes and held at 100 □ 10 psi for at least one minute and until no more fluid drains freely from the compressed lotion package, at which point the container holding the collected lotion is sealed tightly until further analysis. This process is repeated until the desired quantity of lotion for further analysis has been collected. If lotion is collected from more than one package of like wipes, all lotion expressed from all like packages is mixed prior to any analysis.

If lotioned wipes are packaged directly in packaging that is not amenable to the pressure/deformation required by this method, lotioned wipes are removed from packaging and are immediately transferred to a clean rectangular plastic bag and sealed. The rectangular plastic bag is then cut at one corner and placed in the platens of a press and squeezed as specified above.

*Water Content Test Method*

In the Water Content Test Method, a portion of expressed lotion is placed in an oven to facilitate evaporation, and the remaining unevaporated mass is measured. From this, the water content of the starting expressed lotion is calculated.

The Lotion Expression Method is used to express lotion, from which a 5.0 ± 0.1 g aliquot is taken and placed in a 70-mm diameter aluminum weighing boat (such as VWR part number 25433-089, VWR International, Radmor, PA, USA, or equivalent), and the initial mass of the lotion aliquot is determined to at least the nearest 0.001 g. Immediately following weighing, the weighing boat containing the lotion is placed in an oven held at 100 °C for 12 ± 1 hour, at which point the boat containing the unevaporated material remaining from the aliquot is removed, and the mass of unevaporated material is determined to the nearest 0.001 g. The quotient of the unevaporated mass remaining from the lotion aliquot to the initial mass of the lotion aliquot, expressed as a percent to the nearest tenth of a percent, is defined as the Lotion Percent Solids Parameter. The Lotion Percent Solids Parameter is subtracted from 100.0%, and the resulting difference is defined as the Lotion Percent Water Parameter.

*Fluid Retention Value Test Method*

The following procedure can be utilized to determine the fluid retention value of nonwoven substrates of the present disclosure, material layers of the nonwoven substrates of the present disclosure, and pluralities of fibers forming material layers of the nonwoven substrates of the present disclosure.

A sample of about 0.3 g to about 0.4 g of substrate, material layer, or fibers is soaked in a covered container with about 100 ml distilled or deionized water at about 25°C for between about 15 and about 20 hours. The soaked substrate, material layer, or fibers are collected on a filter and transferred to an 80-mesh wire basket supported about 1.5 inches above a 60-mesh screened bottom of a centrifuge tube. The tube is covered with a plastic cover and the sample is centrifuged at a relative centrifuge force of between 2900 x g and 3100 x g for 19 to 21 minutes. The centrifuged substrate, material layer, or fibers are then removed from the basket and weighed. The weighed substrate, material layer, or fibers are dried to a constant weight at 105°C and reweighed. The fluid retention value is calculated as follows:

$$\text{Fluid Retention Value} = (W-D)/D$$

where:

W = wet weight of the centrifuged substrate, material layer, or fibers;
D = dried weight of the substrate, material layer, or fibers; and
W-D = weight of absorbed fluid.

A total of 5 samples are run and the average of the 5 samples is reported as the water retention value for the material in units of g/g or g fluid/g dry substrate, material, or fibers.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A nonwoven substrate (100) comprising:

   a first material layer (102) comprising a first plurality of fibers (104), a second material layer (108) comprising a second plurality of fibers (110), and a third material layer (114) comprising a third plurality of fibers (116);
   a first outer surface (106) formed at least partially of the first material layer (102);
   a second outer surface (112) opposite the first outer surface (106) and formed at least partially of the second material layer (108);
   a plurality of elevated regions (200) disposed on the first outer surface (106);
   a plurality of non-elevated regions (202) disposed on the first outer surface (106) and between the plurality of elevated regions (200);
   wherein fibers of the third plurality of fibers (116) are disposed on the first outer surface (106) in at least some of the plurality of elevated regions (200).

2. The nonwoven substrate (100) of claim 1, wherein the third plurality of fibers (116) are disposed substantially between the first material layer (102) and the second material layer (108) in the non-elevated regions (202).

3. The nonwoven substrate (100) of any of the preceding claims, wherein at least some of the fibers of the third plurality of fibers (116) are intertwined with some of the fibers of the first plurality of fibers (104) in at least some of the elevated regions (200).

4. The nonwoven substrate (100) of any of the preceding claims, wherein the non-elevated regions (202) are interconnected and substantially surround the elevated regions (200).

5. The nonwoven substrate (100) of any of the preceding claims, wherein the third material layer (114) comprises pulp fibers.

6. The nonwoven substrate (100) of claim 5, wherein at least a portion of the pulp fibers are bonded to one another.

7. The nonwoven substrate (100) of any of claims 5 or 6, wherein at least a portion of the pulp fibers are hydrogen bonded to one another.

8. The nonwoven substrate (100) of claim 5, wherein at least a portion of the pulp fibers are not bonded to one another.

9. The nonwoven substrate (100) of any of the preceding claims, wherein the first plurality of fibers (104), the second plurality of fibers (110), and the third plurality of fibers (116) comprise cellulosic fibers.

10. The nonwoven substrate (100) of any of the preceding claims, wherein the first material layer (102) and/or the second material layer (108) comprises carded fibers.

11. The nonwoven substrate (100) of claim 10, wherein the carded fibers have an average length of between about 20 mm and about 80 mm, between about 25 mm and about 75 mm, between about 30 mm and about 70 mm, or between about 35 mm and about 65 mm.

12. The nonwoven substrate (100) of any of the preceding claims, wherein the first material layer (102) and the second material layer (108) are formed of the same fiber type.

13. The nonwoven substrate (100) of any of the preceding claims, wherein the first material layer (102) and the second material layer (108) comprise lyocell fibers.

14. The nonwoven substrate (100) of any of the preceding claims, wherein the second outer surface (112) is substantially free of the third plurality of fibers (116).

15. The nonwoven substrate (100) of any of the preceding claims, wherein the third plurality of fibers (116) forms between about 10 wt.% and about 65 wt.%, between about 20 wt.% and about 50 wt.%, between about 25 wt.% and about 45 wt.%, or between about 30 wt.% and about 42 wt.% of the nonwoven substrate (100).

FIG. 1

FIG. 2

FIG. 3

FIG. 4

Fig. 5

Fig. 6

503

700 701

513

8 | 8

## Fig. 7

701 501

700 700

## Fig. 8

FIG. 9

FIG. 10

500

501

503

513

12                                                                                    12

1200

FIG. 11

500

503

501

1204

1200   1202

502

FIG. 12

FIG. 13

FIG. 14

Fig. 15

Fig. 16

Fig. 17

Fig. 18

Fig. 19

Europäisches
Patentamt

European
Patent Office

Office européen
des brevets

# EUROPEAN SEARCH REPORT

**Application Number**

EP 24 18 8914

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/358101 A1 (KANYA KEVIN RONALD [US] ET AL) 4 December 2014 (2014-12-04) | 1-4, 10-12, 14,15 | INV. D04H1/425 D04H1/4258 |
| A | * figure 1 * <br> * paragraphs [0026] - [0027], [0029] - [0030], [0032] * <br> * paragraphs [0035], [0040] * <br> * paragraph [0037] * <br> * paragraph [0024] * | 5-9,13 | D04H1/495 D04H1/498 B32B3/30 B32B5/02 B32B5/26 |
| | ----- | | ADD. |
| X | US 2017/258647 A1 (ORR JILL MARLENE [US] ET AL) 14 September 2017 (2017-09-14) | 1,2,4, 10-12, 14,15 | A61F13/511 A61F13/537 A61F13/514 |
| A | * paragraphs [0237] - [0246]; figure 13 * <br> * paragraphs [0330] - [0331] * | 3,5-9,13 | |
| | ----- | | |
| X | US 2006/084344 A1 (BONNEH ACHAI [IL]) 20 April 2006 (2006-04-20) | 1-4,12, 15 | |
| A | * figure 1 * <br> * paragraphs [0023], [0025], [0026] * | 5-11,13, 14 | |
| | ----- | | TECHNICAL FIELDS SEARCHED (IPC) |
| X | WO 2022/016051 A1 (BERRY GLOBAL INC [US]) 20 January 2022 (2022-01-20) | 1-8, 10-12, 14,15 | D04H B32B |
| A | * page 18, line 32 - page 19, line 9; figures 3A-3B * <br> * page 16, lines 26-28 * <br> * page 16, line 31 - page 17, line 32 * <br> * page 11, lines 7-10 * <br> * page 9, line 36 * | 9,13 | A61F |
| | ----- | | |

The present search report has been drawn up for all claims

| Place of search | Date of completion of the search | Examiner |
|---|---|---|
| Munich | 23 October 2024 | Beckert, Audrey |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 8914

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2024

| Patent document cited in search report | | Publication date | Patent family member(s) | | Publication date |
|---|---|---|---|---|---|
| US 2014358101 | A1 | 04-12-2014 | CN | 105229212 A | 06-01-2016 |
| | | | EP | 3004444 A1 | 13-04-2016 |
| | | | JP | 6185155 B2 | 23-08-2017 |
| | | | JP | 2016524659 A | 18-08-2016 |
| | | | US | 2014358101 A1 | 04-12-2014 |
| | | | WO | 2014193660 A1 | 04-12-2014 |
| US 2017258647 | A1 | 14-09-2017 | US | 2017258647 A1 | 14-09-2017 |
| | | | WO | 2017156203 A1 | 14-09-2017 |
| US 2006084344 | A1 | 20-04-2006 | US | 2006084344 A1 | 20-04-2006 |
| | | | WO | 2006040752 A1 | 20-04-2006 |
| WO 2022016051 | A1 | 20-01-2022 | DK | 4181849 T3 | 13-05-2024 |
| | | | EP | 4181849 A1 | 24-05-2023 |
| | | | ES | 2981266 T3 | 08-10-2024 |
| | | | FI | 4181849 T3 | 21-05-2024 |
| | | | PL | 4181849 T3 | 02-09-2024 |
| | | | US | 2022016867 A1 | 20-01-2022 |
| | | | WO | 2022016051 A1 | 20-01-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7666827 B
- US 7005557 B
- US 8221774 B
- US 6607696 B
- US 6602466 B
- US 7922984 B
- US 20140005020
- US 9421137 B
- US 20170191198 A
- US 5628097 A, Benson
- US 20160136014 A, Arora

- WO 2023022979 A
- US 5599335 A, Goldman
- EP 1447066 A, Busam
- WO 9511652 A, Tanzer
- US 20080312622 A1, Hundorf
- WO 2012052172 A, Van Malderen
- US 7838722 B, Blessing
- US 9072634 B
- US 8206533 B, Hundorf
- US 9789011 B